(19) Europäisches Patentamt
European Patent Office
Office européen des brevets

(11) Veröffentlichungsnummer : **0 103 543**
**B1**

(12) **EUROPÄISCHE PATENTSCHRIFT**

(45) Veröffentlichungstag der Patentschrift : **30.09.87**

(21) Anmeldenummer : **83810400.8**

(22) Anmeldetag : **02.09.83**

(51) Int. Cl.⁴ : **C 07 D 401/12**, A 01 N 47/36// C07D213/73, C07D213/71, C07D213/74, C07D213/72

(54) **Neue Sulfonylharnstoffe.**

(30) Priorität : **08.09.82 CH 5337/82**
**28.04.83 CH 2283/83**

(43) Veröffentlichungstag der Anmeldung : **21.03.84 Patentblatt 84/12**

(45) Bekanntmachung des Hinweises auf die Patenterteilung : **30.09.87 Patentblatt 87/40**

(84) Benannte Vertragsstaaten : **BE CH DE FR GB IT LI NL**

(56) Entgegenhaltungen :
**EP-A- 0 023 422**
**EP-A- 0 030 433**
**EP-A- 0 035 893**
**EP-A- 0 044 807**
**EP-A- 0 044 808**
**EP-A- 0 044 809**
**EP-A- 0 056 969**
**EP-A- 0 070 802**
**EP-A- 0 072 347**
**EP-A- 0 082 108**
**EP-A- 0 084 020**
**EP-A- 0 085 028**
**EP-A- 0 094 790**
**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Klybeckstrasse 141**
**CH-4002 Basel (CH)**

(72) Erfinder : **Föry, Werner, Dr.**
**Benkenstrasse 65**
**CH-4054 Basel (CH)**
Erfinder : **Gass, Karl**
**Blumenrain 4**
**CH-4312 Magden (CH)**
Erfinder : **Meyer, Willy**
**Talweg 49**
**CH-4125 Riehen (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die vorliegende Erfindung betrifft neue, herbizid wirksame und planzenwuchsregulierende N-Pyridinsulfonyl-N'-pyrimidinyl- und -triazinylharnstoffe, Verfahren zu ihrer Herstellung, sie als Wirkstoffe enthaltende Mittel, sowie deren Verwendung zum Bekämpfen von Unkräutern, vor allem selektiv in Nutzpflanzenkulturen oder zum Regulieren und Hemmen des Pflanzwachstums.

Die erfindungsgemässen N-Pyridinsulfonyl-N'-pyrimidinyl- und -triazinylharnstoffe entsprechen der Formel I

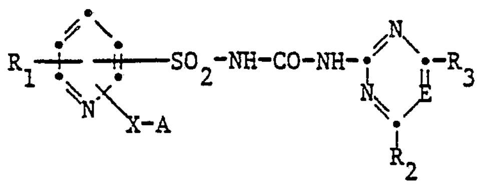

(I)

worin

A einen $C_3$-$C_6$-Alkinylrest, einen durch Halogen, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Halogenalkoxy, $C_1$-$C_4$-Halogenalkylthio, $C_1$-$C_4$-Halogenalkylsulfinyl oder $C_1$-$C_4$-Halogenalkylsulfonyl substituierten $C_1$-$C_6$-Alkylrest oder einen gegebenfalls durch die aufgezählten Reste substituierten $C_2$-$C_6$-Alkenylrest, einen Phenylrest, der unsubstituiert oder durch Halogen, Cyano, Nitro, $C_1$-$C_4$-Alkyl, $C_1$-$C_4$-Halogenalkyl, einen Rest —X—$C_1$-$C_4$-Alkyl, $C_1$-$C_4$-Alkoxycarbonyl, Amino, Mono- oder Di-($C_1$-$C_4$-alkyl) amino, Carbamoyl, Mono- oder Di-($C_1$-$C_4$-alkyl) carbamoyl, Sulfamoyl, Mono- oder Di-$C_1$-$C_4$-alkyl) sulfamoyl substituiert ist, oder der Rest

X—A bildet einen Aminorest —$NR_6R_7$,

E die Methingruppe oder Stickstoff,

$R_1$ Wasserstoff, Halogen, einen $C_1$-$C_4$-Alkyl-, $C_1$-$C_4$-Alkoxy-, $C_1$-$C_4$-Halogenalkyl-, $C_1$-$C_4$-Halogenalkoxy, $C_2$-$C_5$-Alkoxyalkoxy-, $C_1$-$C_5$-Alkylthio-, $C_1$-$C_5$-Alkylsulfinyl- oder einen $C_1$-$C_5$-Alkylsulfonylrest,

$R_2$ gegebenenfalls durch 1-3 Halogenatome substituiertes $C_1$-$C_3$-Alkyl, oder $C_1$-$C_3$-Alkoxy,

$R_3$ Wasserstoff, Halogen, eine Aminogruppe —$NR_4R_5$, gegebenenfalls durch 1-3 Halogenatome substituiertes $C_1$-$C_3$-Alkyl oder gegebenenfalls durch Mèthoxy, Aethoxy oder 1-3 Halogenatome substituiertes $C_1$-$C_4$-Alkoxy,

$R_4$ Wasserstoff oder Methyl,

$R_5$ Wasserstoff, $C_1$-$C_2$-Alkyl oder Methoxy

$R_6$ und $R_7$ je einzeln Wasserstoff, $C_1$-$C_4$-Alkyl, $C_3$-$C_6$-Alkenyl, $C_3$-$C_6$-Alkinyl, $C_3$-$C_6$-Alkoxyalkyl, $C_1$-$C_4$-Cyanoalkyl oder

$R_6$ und $R_7$ zusammen mit dem sie verbindenden Stickstoffatom auch einen gesättigten, 5-6-gliedrigen Heterocyclus, der auch Sauerstoff, Schwefel oder einen Rest —$NR_8$- enthalten kann,

$R_8$ Wasserstoff, $C_1$-$C_4$-Alkyl, oder Benzyl, und

X Sauerstoff, Schwefel, eine Sulfinyl- oder Sulfonylbrücke bedeuten, mit der Massgabe, dass wenn —X—A einen $C_3$-$C_4$-Alkenyl-sulfid-, -sulfinyl- oder -sulfonylrest und $R_1$ gleichzeitig Wasserstoff, Halogen, Methyl, Methoxy, Trifluoromethyl, Nitro, Cyano oder Amino bedeuten, die Reste $R_2$ auf $C_1$-$C_2$-Alkyl, $C_1$-$C_2$-Alkoxy oder Methoxymethyl und $R_3$ auf Methyl oder Methoxy beschränkt sind,

sowie den Salzen dieser Verbindungen.

Harnstoffverbindungen, Triazinverbindungen und Pyrimidinverbindungen mit herbizider Wirkung sind allgemein bekannt. Kürzlich wurden Pyridyl-sulfamoyl-heterocyclyl-aminocarbamoylverbindungen mit herbizider und pflanzenwuchsregulierender Wirkung, beispielsweise in den europäischen Patentpublikationen Nr. 13 480 und 35 893 beschrieben.

Die EP-A-30 433 beschreibt herbizid- und den Pflanzenwuchs regulierende N-Phenylsulfonyl- und N-Pyridinsulfonyl-N'-pyrimidinyl und -triazinylharnstoffe, welche im Pyrimidin-, respektive im Triazinring durch eine Amidogruppe substituiert sind.

Aus den publizierten europäischen Patentpublikationen EP-A-23 422, 44 807, 44 808, 44 809, 56 969, 70 802, 72 347, 82 108, 84 020 und 85 028 sind ferner Verbindungen bekannt geworden die sich von den erfindungsgemässen Pyridinsulfonylharnstoffen dadurch unterscheiden, dass sie anstelle des Pyridinringes einen Phenylring besitzen. Die erfindungsgemässen Pyridinsulfonylharnstoffe sind den Kulturen gegenüber toleranter und eignen sich besser zur Kontrolle von Unkräutern in Kulturen von z. B. Weizen, Mais und Soja, als die entsprechenden Phenylsolfonylharnstoff-Homologen.

In den Definitionen ist unter Alkyl geradkettiges oder verzweigtes Alkyl zu verstehen : z. B. Methyl, Aethyl, n-Propyl, i-Propyl, die vier isomeren Butyl, n-Amyl, i-Amyl, 2-Amyl, 3-Amyl, n-Hexyl oder i-Hexyl.

Unter Alkoxy ist zu verstehen : Methoxy, Aethoxy, n-Propyloxy, i-Propyloxy und die vier isomeren Butyloxyreste, insbesondere aber Methoxy, Aethoxy oder i-Propoxy.

Beispiele für Alkylthio sind Methylthio, Aethylthio, n-Propylthio, i-Propylthio und n-Butylthio, insbesondere aber Methylthio und Aethylthio.

Beispiele für Alkenylreste sind Vinyl, Allyl, Isopropenyl, 1-Propenyl, 1-Butenyl, 2-Butenyl, 3-Butenyl, 1-Isobutenyl, 2-Isobutenyl, 1-Pentenyl, 2-Pentenyl, 3-Pentenyl und 4-Pentenyl, insbesondere aber Vinyl, Allyl und 4-Pentenyl.

Beispiele für Alkylsulfinyl sind Methylsulfinyl, Aethylsulfinyl, n-Propylsulfinyl und n-Butylsulfinyl, insbesondere aber Methylsulfinyl und Aethylsulfinyl.

Beispiele für Alkylsulfonyl sind Methylsulfonyl, Aethylsulfonyl, n-Propylsulfonyl und n-Butylsulfonyl, insbesondere aber Methylsulfonyl und Aethylsulfonyl.

Unter Halogen in den Definitionen sowie in Halogenalkyl, -alkoxy, -alkylsulfinyl, -alkylsulfonyl und -alkylthio sind Fluor, Chlor und Brom, vorzugsweise jedoch Fluor und Chlor zu verstehen.

Entsprechend sind unter Halogenalkyl bzw. Halogenalkylteilen von oben definierten Substituenten beispielweise zu verstehen : Chlormethyl, Fluormethyl, Difluormethyl, Trifluormethyl, 2-Chloräthyl, 2,2,2-Trifluoräthyl, 1,1,2,2-Tetrafluoräthyl, Pentafluoräthyl, 1,1,2-Trifluor-2-chloräthyl, 2,2,2-Trifluor-1,1-dichloräthyl, Pentachloräthyl, 3,3,3-Trifluorpropyl, 2,3-Dichlorpropyl, 1,1,2,3,3,3-Hexafluorpropyl, insbesondere aber Chlormethyl, Difluormethyl und Trifluormethyl.

Alkinylresten in den Definitionen der obigen Symbole entsprechen in der Regel Propargyl, 2-Butinyl, 3-Butinyl, sowie isomere Pentinyl- oder Hexinylreste, vorzugsweise ist der Alkinylrest jedoch durch Propargyl oder 2- oder 3-Butinyl verkörpert.

Die Erfindung umfasst ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für Salzbildung geeignete Amine sind primäre, sekundäre und tertiäre aliphatische und aromatische Amine wie Methylamin, Aethylamin, Propylamin, i-Propylamin, die vier isomere Butylamine, Dimethylamin, Diäthylamin, Diäthanolamin, Dipropylamin, Diisopropylamin, Di-n-butylamin, Pyrrolidin, Piperidin, Morpholin, Trimethylamin, Triäthylamin, Tripopylamin, Chinuclidin, Pyridin, Chinolin, und i-Chinolin, insbesondere aber Aethyl-, Propyl-, Diäthyl- oder Triäthylamin, vor allem aber iso-Propylamin und Diäthanolamin.

Beispiele für quaternäre Ammoniumbasen sind im allgemeinen die Kationen von Halogenammoniumsalzen, z. B. das Tetramethylammoniumkation, das Trimethylbenzylammoniumkation, das Triäthylbenzylammoniumkation, das Tetraäthylammoniumkation, das Trimethyläthylammoniumkation, aber auch das Ammoniumkation.

Von den erfindungsgemässen Verbindungen der Formel I sind die Verbindungen bevorzugt, in denen

a) $R_1$ Wasserstoff bedeutet,
b) $R_1$ Wasserstoff und X Sauerstoff oder Schwefel bedeuten,
c) diejenigen welche der folgenden Formel entsprechen.

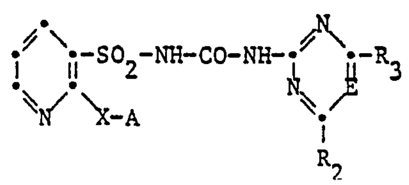

d) denjenigen Verbindungen obigen Formel, in denen X Sauerstoff bedeutet,
e) die N-Pyridinsulfonyl-N'-pyrimidinylharnstoffe der Formel

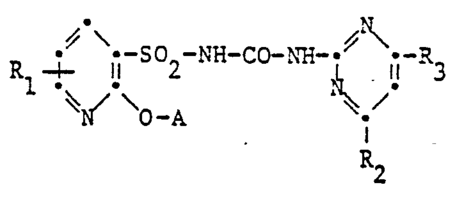

f) die N-Pyridinsulfonyl-N'-triazinylharnstoffe der Formel

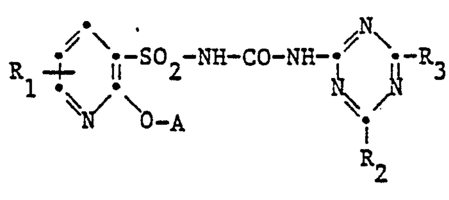

g) ferner die N-Pyridin sulfonylharnstoffe der folgenden Formel

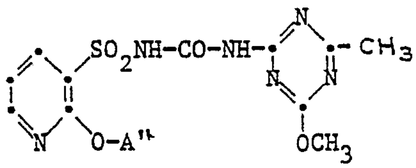

in der A″ $C_3$-$C_4$-Alkenyl oder $C_3$-$C_4$-Alkoxyalkyl bedeuten.

In den obigen Formeln haben A, E, $R_1$, $R_2$, $R_3$ und X die unter Formel I gegebene Bedeutung.

Die Herstellung der Verbindungen der Formel I erfolgt in einem inerten organischen Lösungsmittel.

Nach einem ersten Verfahren werden die N-Pyridinsulfonyl-N'-pyrimidinyl- oder -triazinylharnstoffe der Formel I hergestellt, indem man ein Pyridylsulfonamid der Formel II

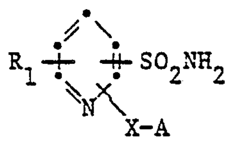
(II)

worin A, $R_1$ und X die unter Formel I gegebene Bedeutung haben, in Gegenwart einer Base mit einem N-Pyrimidinyl- oder N-Triazinylcarbamat der Formel III umsetzt,

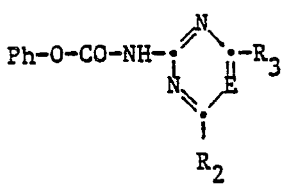
(III)

worin $R_2$, $R_3$ und E die unter Formel I gegebene Bedeutung haben, und Ph einen gegebenenfalls durch Halogen oder Alkyl substituierten Phenylrest bedeutet.

Nach einem zweiten Verfahren gelangt man zu den N-Pyridinsulfonyl-N'-pyrimidinyl- und -triazinylharnstoffe der Formel I, indem man ein Pyridinsulfonylisocyanat der Formel IV

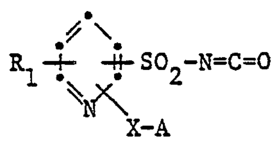
(IV)

worin A, $R_1$ und X die unter Formel I gegebene Bedeutung haben, gegebenenfalls in Gegenwart einer Base, mit einem Amin der Formel V umsetzt,

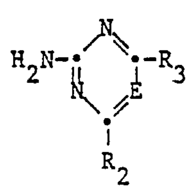
(V)

worin E, $R_2$ und $R_3$ die unter Formel I gegebene Bedeutung haben.

Nach einem dritten Verfahren gelingt es die N-Pyridinsulfonyl-N'-pyrimidinyl- und -triazinylharnstoffe der Formel I herzustellen, indem man ein Pyridinsulfonylcarbamat der Formel VI

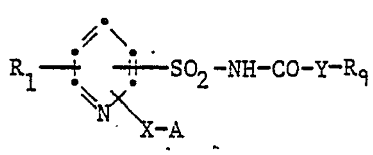
(VI)

worin $R_q$ Phenyl unsubstituiert oder durch Halogen oder $C_1$-$C_4$-Alkyl substituiert, $C_1$-$C_4$-Alkyl oder $C_2$-$C_4$-Alkoxyalkyl und Y Sauerstoff oder Schwefel bedeutet und A, $R_1$ und X die unter Formel I gegebene Bedeutung haben, in Gegenwart einer Base als säurebindendes Mittel mit einem Amin der Formel V umsetzt.

Die erhaltenen Harnstoffe der Formel I können gewünschtenfall mittels Aminen, Alkalimetall- oder Erdalkalimetallhydroxiden oder quaternären Ammoniumbasen in Additionssalze übergeführt werden. Dieses geschieht beispielsweise durch Umsetzen mit der äquimolaren Menge Base und Verdampfen des Lösungsmittels.

Die Ausgangsmaterialien der Verbindungen II, IV und VI sind neue Verbindungen. Sie und ihre Herstellung sind ebenfalls Gegenstand dieser Erfindung.

Die Pyridinsulfonamide der Formel II werden hergestellt, indem man ein entsprechend substituiertes Pyridinamin durch Diazotierung und Austausch der Diazogruppe mit Schwefeldioxid in Gegenwart eines Katalysators ; z. B. Kupfer-I-chlorid in Salzsäure oder Essigsäure und Umsetzen des erhaltenen Pyridinsulfonylchlorides mit Ammoniak. Die entsprechenden Pyridinamine sind bekannt oder nach bekannten Methoden herstellbar. In bestimmten Fällen, wenn z. B. eine aktivierte Substitutionsposition vorliegt, ist eine direkte Sulfochlorierung des Pyridinringes möglich. Man arbeitet dann mit einem Ueberschuss an Chlorschwefelsäure und kommt zum entsprechenden Pyridinsulfochlorid. Aehnliche Umsetzungen von Alkoxyaniliden sind beispielsweise aus der publizierten Europaïschen Patentanmeldung Nr. 44 807 beschrieben.

Die Pyridinsulfonylamide der Formel II können in Abwandlung einer im J. Med. Chem. 23, 1376 (1980) beschriebenen Methode auch erhalten werden durch Chlorierung mittels Chlorgas in wässriger Lösung von geeignet substituiertem Pyridinmerkaptan. Man erhält so ein Pyridinsulfochlorid welches mit Ammoniak zum Sulfonamid umgesetzt wird.

Durch Modifikation eines in den Ann. Pharm. Fr. 31, 467 (1973) beschriebenen Verfahrens kann man einen geeignet substituierten Pyridinring gegebenenfalls in Gegenwart eines inerten Lösungsmittels zuerst mit Oleum und dann mit Phosphorpentachlorid behandeln, um zum Pyridinsulfochlorid zu kommen welches dann mit Ammoniak zum Pyridinsulfonamid der Formel II umgesetzt wird.

Die neuen Pyridylsulfonamide der Formel II können ferner erhalten werden, indem man ein am geeigneter Stelle halogeniertes Pyridylsulfonamid in Gegenwart einer Base mit einem Alkohol, Thiol, einer Sulfinyl- oder Sulfonylverbindung umsetzt, entsprechend der Gleichung

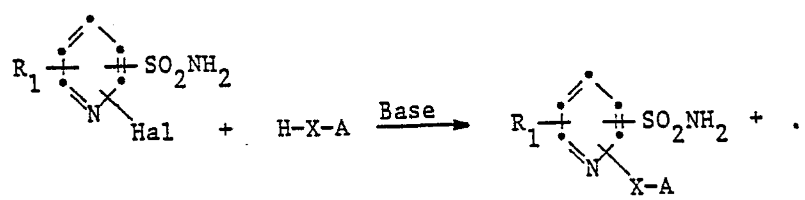


In diesen Formeln haben A, $R_1$ und X die unter Formel I gegebene Bedeutung. Einzelheiten zu solchen Umsetzungen sind z. B. dem J. Pharm. Belg. 35, 98 (1980) zu entnehmen.

Die neuen Pyridylsulfonamide der Formel II werden ferner erhalten, wenn man an ein Hydroxy- oder Mercaptopyridylsulfonamid in Gegenwart eines inerten Lösungsmittels und einer Base als säurebindendes Mittel mit einen Halogenid umsetzt, entsprechend der Gleichung

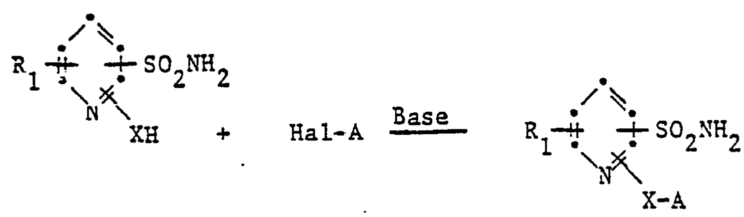


In diesen Formeln haben A, $R_1$ und X die unter Formel I gegebene Bedeutung während Hal ein Halogenatom, insbesondere Chlor oder Brom Bedeutet. Solche Umsetzungen sind beispielsweise in der publizierten Europäischen Patentanmeldung Nr. 44 807 beschrieben.

Ferner ist es möglich die Pyridylsulfonamide, in denen X die Sulfinyl- oder Sulfonylbrücke bedeutet, durch Oxidation der entsprechenden Thio-Verbindung zu erhalten. Solche Oxydationen sind beispielsweise in der publizierten Europäischen Patentanmeldung Nr. 35 893 beschrieben.

Die Sulfonylisocyanate der Formel IV können durch Umsetzung der Sulfonamide der Formel II mit Phosgen in Anwesenheit von Butylisocyanat in einem chlorierten Kohlenwasserstoff als Lösungsmittel bei Rückflusstemperatur erhalten werden. Aehnliche Herstellungsmethoden sind in « Neuere Methoden der präparativen organischen Chemie », Band VI, 211-229, Verlag Chemie, Weinheim, 1970, beschrieben.

Die Pyridinsulfon ylcarbamate der Formel VI werden durch Umsetzung der Sulfonamide der Formel II mit Diphenylcarbonat oder Chlorameisensäurephenylester in Gegenwart einer Base hergestellt. Aehnliche Verfahren sind in der japanischen Patentschrift 61 169 erwähnt.

Die als Ausgangsmaterialien verwendeten Aminopyrimidine und -triazine der Formel V sowie entsprechende Phenylcarbamate der Formel III sind entweder seit langem bekannt oder in der schweizerischen Patentanmeldung Nr. 3527/82-8 beschrieben oder sie lassen sich nach bekannten

Methoden aus dort beschriebenen Verbindungen erhalten.

Diese Umsetzungen zu Verbindungen der Formel I werden vorteilhafterweise in aprotischen, inerten, organischen Lösungsmitteln vorgenommen wie Methylenchlorid, Tetrahydrofuran, Acetonitril, Dioxan, Toluol.

Die Reaktionstemperaturen liegen vorzugsweise zwischen —20° und + 120° C. Die Umsetzungen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base oder Isocyanat als Reaktionskatalysator verkürzt werden.

Die Endprodukten können durch Einengen und/oder Verdampfen des Lösungsmittels isoliert und durch Umkristallisieren oder Zerreiben des festen Rückstandes in Lösungsmitteln in denen sich nicht gut lösen, wie Aether, aromatischen Kohlenwasserstoffen oder chlorierten Kohlenwasserstoffen gereinigt werden.

Die Wirkstoffe der Formel I sind stabile Verbindungen. Ihre Handhabung bedarf keine vorsorglicher Massnahme.

Bei geringeren Aufwandmengen zeichnen sich die Verbindungen der Formel I durch gute selektivwuchshemmende und selektiv-herbizide Eigenschaften aus, die sie ausgezeichnet zum Einsatz in Kulturen von Nutzpflanzen, insbesondere in Getreide, Baumwolle, Soja, Mais und Reis, befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d. h. sie werden von der Pflanze aufgenommen und an andere Stellen transprotiert, wo sie dann zur Wirkung kommen. So gelingt es beispielsweise, durch Oberflächenbehandlung perenierende Unkräuter bis in die Wurzeln zu schädigen. Die neuen Verbindungen der Formel I wirken bereits bei — im Vergleich zu anderen Herbiziden und Wuchsregulatoren — sehr geringen Aufwandmengen.

Die Verbindungen der Formel I haben ausserdem starke pflanzenwuchsregulierende, insbesondere pflanzenwuchshemmende, Eigenschaften. Es werden sowohl Monokotyledonen als auch Dikotyledonen in ihrem Wachstum beeinträchtigt.

So können z. B. die in der Landwirtschaft in tropischen Gegenden häufig als « cover crops » (Bodendecker) angepflanzten Leguminosen durch die Verbindungen der Formel I in ihrem Wachstum selektiv gehemmt werden, so dass zwar die Bodenerosion zwischen den Kulturpflanzen verhindert wird, die « cover crops » jedoch nicht zur Konkurrenz für die Kultur werden können.

Bei grösseren Aufwandmengen werden alle getesteten Pflanzen in ihrer Entwicklung so geschädigt, dass sie absterben.

Die Erfindungen betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen : aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dikotylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl ; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalische Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch. Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z. B. die Na- oder K-Salze der Oel- oder Stearinsäure, oder von natürlichen Fettsäuregemischen, die z. B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazol-derivate oder Alkalarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z. B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z. B. Salze des Phosphorsäureester eines p-Nonylphenol-(4-14)-Aethylenoxid-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten Können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispielen nichtionische Tenside seien Nonylphenolpolyäthoxyäthanole, Ricinusölpolyglykoläther, Polypropylenpolyäthylenoxid-Addukte, Tributylphenoxypolyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z. B. das Stearyltrimethylammoniumchlorid oder das Benzyldi(2-chloräthyl)-äthylammoniumbromid.

Die in der Formulierungstechnik gebräuchlichen Tenside sind u. a. in folgenden Publikationen beschrieben :

« Mc Cutcheon's Detergents and Emulsifiers Annual » MC Publishing Corp., Ringwood, New Jersey, 1979 ;

Sisely and Wood, « Encyclopedia of Surface Active Agents », Chemical Publishing Co. Inc., New York, 1964.

Die pestiziden Zubereitungen enthalten in der Regel 0,1 bis 95 %, insbesondere 0,1 bis 80 %, Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen : (% = Gewichtsprozent)

Emulgierbare Konzentrate

| | | |
|---|---|---|
| Aktiver Wirkstoff | 1 bis 20 %, bevorzugt | 5 bis 10 % |
| oberflächenaktives Mittel | 5 bis 30 %, vorzugsweise | 10 bis 20 % |
| flüssiges Trägermittel | 50 bis 94 %, vorzugsweise | 70 bis 85 %. |

Stäube

| | | |
|---|---|---|
| Aktiver Wirkstoff | 0,1 bis 10 %, vorzugsweise | 0,1 bis 1 % |
| festes Trägermittel | 99,9 bis 90 %, vorzugsweise | 99,9 bis 99 %. |

Suspension-Konzentrate

| | |
|---|---|
| Aktiver Wirkstoff | 5 bis 75 %, vorzugsweise 10 bis 50 % |
| Wasser | 94 bis 25 %, vorzugsweise 90 bis 30 % |
| oberflächenaktives Mittel | 1 bis 40 %, vorzugsweise 2 bis 30 %. |

Benetzbare Pulver

| | |
|---|---|
| Aktiver Wirkstoff | 0,5 bis 90 %, vorzugsweise 1 bis 80 % |
| oberflächenaktives Mittel | 0,5 bis 20 %, vorzugsweise 1 bis 15 % |
| festes Trägermittel | 5 bis 95 %, vorzugsweise 15 bis 90 %. |

Granulate

| | |
|---|---|
| Aktiver Wirkstoff | 0,5 bis 30 %, vorzugsweise 3 bis 15 %. |
| festes Trägermittel | 99,5 bis 70 %, vorzugsweise 97 bis 85 %. |

Während als Handelsware eher konzentrierte Mittel bevorzugt werden, werwendet der Endverbraucher in der Regel verdünnte Mittel. Die Anwendungsformen können bis hinab zu 0,001 % an Wirkstoff verdünnt werden. Die Aufwandmengen betragen in der Regel 0,001 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

In den folgenden Beispielen sind die Temperaturen in Celsiusgraden °C, die Drücke in Millibar mb angegeben.

Beispiel 1 : Verfahren zur Herstellung von N-[2-(2-Methoxyäthoxy)-3-pyridinsulfonyl]-N'-[4-methoxy-6-methyl-1,3,5-triazin-2-yl]-harnstoff der Formel

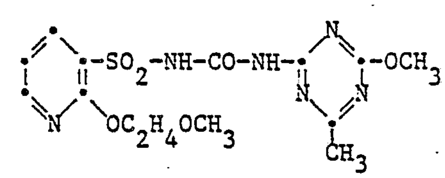

Zu einem Gemisch von 4,64 g 2-(2-Methoxyäthoxy)-3-pyridinsulfonamid in 3,1 ml 1,8-Diazabicyclo [5,4,0] undec-7-en in 50 ml absolutem Dioxan werden bei einer Temperatur von maximal 22 °C 5,2 g N-(4-Methoxy-6-methyl-1,3,5-triazin-2-yl)-phenylcarbamat gegeben. Das gemisch wird dann eine Stunde bei Raumtemperatur gerührt, am Vakuum eingeengt und mit 12 ml 2 N wässariger Salzsäure verrührt und filtriert. Der Rückstand wird mit Wasser gewaschen und getrocknet. Man erhält so 7,9 g des obigen Harnstoffes, welcher einen Schmelzpunkt von 112-114 °C aufweist.

Das als Ausgangsprodukt benötigte 2-(2-Methoxyäthoxy)-3-pyridylsulfonamid wird wie folgt hergestellt :

Zu 25 ml Methylcellosolve gibt man portionenweise, unter Stickstoffatmosphäre während ca. 15 Minuten 4,36 g einer 55 %igen Natriumdispersion in Oel. Zu diesem Dispersion tropft man während 5 Minuten unter Rühren eine Lösung von 9,6 g 2-Chlor-3-pyridylsulfonamid in 25 ml Methylcellosolve. Das Reaktionsgemisch wird dann während einer Stunde bei Rückflusstemperatur gerührt und am Vakuum eingeengt. Der Rückstand bei einer Temperatur von unter 15 °C mit ca. 25 ml wässriger Salzsäure auf pH 2 angesäuert, verrührt und filtriert. Man erhält so 11,3 g des gewünschten 2-(2-Methoxyäthoxy)-3-pyridylsulfonamides, welches aus Acetonitril umkristallisiert, einen Schemlzpunkt von 102-103 °C aufweist.

Beispiel 2 : Verfahren zur Herstellung von N-[2-Dimethylamino-3-pyridinsulfonyl]-N'-[4-methoxy-6-methyl-1,3,5-triazin-2-yl]-harnstoff der Formel

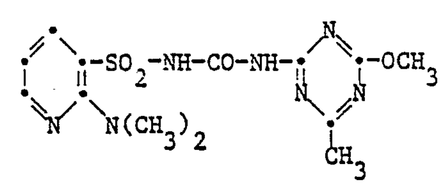

Zu einem Gemisch von 2,41 g 2-Dimethylamino-3-pyridinsulfonamid und 1,9 ml 1,8-Diazabicyclo [5.4.0] undec-7-en in 30 ml absolutem Dioxan werden bei Raumtemperatur 3,28 g N-(4-Methoxy-6-methyl-1,3,5-triazin-2-yl) phenylcarbamat gegeben. Das Gemisch wird eine Stunde bei dieser Temperatur gerührt, dann am Vakuum eingeengt, mit 10 ml 1 N wässeriger Salzsäure verrührt und filtriert. Der Rückstand wird mit Wasser und Aether gewaschen und getrocknet. Man erhält so 3,94 g des obigen Harnstoffes mit einem Schmelzpunkt von 173-175 °C.

Das als Ausgangsprodukt benötigte 2-Dimethylamino-3-pyridinsulfonamid wird wie folgt hergestellt :

In einem Druckgefäss, das 6,73 g 2-Chlor-3-pyridinsulfonamid in 60 ml absolutem Tetrahydrofuran enthält werden bei 0 °C 6.32 g Dimethylamin eingepresst. Der Inhalt wird 75 Minuten bei 60° gerührt, dann auf Raumtemperatur gekühlt, filtriert, das Filtrat am Vakuum eingeengt. Der Rückstand wird mit Aether/Petroläther (1 : 1) verrührt und filtriert. Man erhält so 6,32 g des obigen Sulfonamids mit einem Schmelzpunkt von 98°-100 °C.

Beispiel 3 : Verfahren zur Herstellung von N-[2-Phenoxy-3-pyridinsulfonyl]-N'-[4-methoxy-6-methyl-1,3,5-triazin-2-yl]-harnstoff der Formel

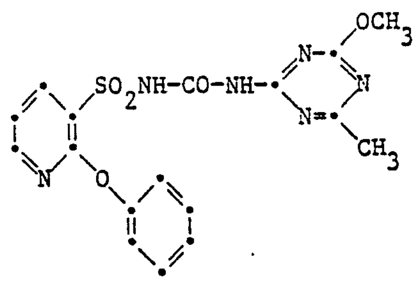

a) Zu einem Gemisch von 2,00 g 2-Phenoxy-3-pyridinsulfonamid, 1.4 ml 1,8-Diazabicyclo [5.4.0] undec-7-en in 25 ml absolutem Acetonitril werden bei einer Temperatur von 25 °C 2,08 g N-(4-Methoxy-6-methyl-1,3,5-triazin-2-yl)-phenylcarbamat gegeben. Das Gemisch wird während 20 Stunden bei Raumtemperatur gerührt und dann auf 150 ml HCl in Eis gegossen.

Der erhaltene Niederschlag wird filtriert, mit Wasser gewaschen, getrocknet und aus Essigester kristallisiert.

Man erhält so 2,0 g des obigen Harnstoffes, welcher einen Schmelzpunkt von 181-182 °C aufweist.

Das als Ausgangsprodukt benötigte 2-Phenoxy-3-pyridylsulfonamid wird wie folgt hergestellt :

b) Zu einer Lösung von 20,7 g Phenol in 150 ml Dimethylsulfoxid werden 14,0 g KOH-Pulver (88 %-ig) gegeben und 1 1/2 Stunden gerührt. Dann gibt man eine Lösung von 19,26 g 2-Chlor-3-pyridylsulfonamid in 50 ml Dimethylsulfoxid und 0,1 g 18-Crown-6 zu und rührt anschliessend 44 Stunden bei 130 °C. Das Reaktionsgemisch wird auf ein Gemisch von 2 N Salzsäure und Eis gegossen und gut gerührt. Der erhaltene Niederschlag wird abfiltriert, gut mit Wasser gewaschen, getrocknet und aus Essigester umkristallisiert.

Man erhält so 17,5 g des gewünschten 2-Phenoxy-3-pyridylsulfonamides, welches aus Essigester umkristallisiert, einen Schmelzpunkt bzw. 176-178 °C aufweist.

Beispiel 4 : Verfahren zur Herstellung von N-[2-Allyloxy-3-pyridinsulfonyl]-N'-[4,6-dimethoxy-1,3,5-triazin-2-yl]-harnstoff der Formel

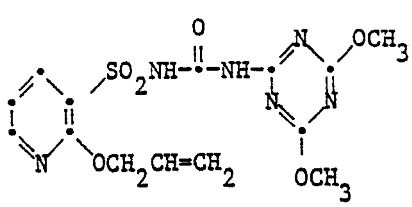

a) Zu einer Mischung von 2,03 g 2-Amino-4,6-dimethoxy-1,3,5-triazin und 0,09 ml Triäthylamin in 30 ml absolutem Dioxan werden bei 60 °C innerhalb 15 Minuten 4,83 g N-2-Allyloxy-3-pyridin-sulfonylphenylcarbamat in 30 ml Dioxan zugetropft. Die Reaktionsmischung wird während 1 1/2 Stunden bei 70 °C gerührt, warm filtriert und bei 50 °C aus Vakuum eingedampft. Der ölige Rückstand wird dann mit Aether verrieben und filtriert. Man erhält so 3,28 g des gewünschten Sulfonylharnstoffs, welcher einen Schmelzpunkt von 152°-154 °C aufweist.

Das als Ausgangsprodukt benötigte N-2-Allyloxy-3-pyridinsulfonylphenylcarbamat der Formel

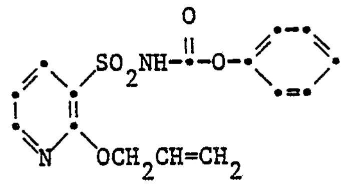

wird wie folgt hergestellt :

b) Zu einer Suspension von 0,87 g Natriumhydrid (55 %) in 10 ml absolutem Dimethylformamid (DMF) wird bei max. 20 °C innerhalb 5 Minuten eine Lösung von 4,7 g Diphenylcarbonat in 30 ml DMF zugetropft. In dieser Reaktionsmischung wird nun während 20 Minuten bei maximal 20 °C eine Lösung von 4,28 g 2-Allyloxy-3-pyridinsulfonamid in 20 ml DMF zugetropft und während 1 Stunde bei dieser Temperatur noch gerührt. Die Reaktionslösung wird in einer Mischung von 130 ml Essigester, 130 ml Eis/Wasser und 19,2 ml 2 N Salzsäure aufgenommen, die organische Phase abgetrennt, vier mal mit kaltem Wasser gewaschen, über Natriumsulfat getrocknet und eingedampft. Der Rückstand wird mit Aether verrieben, filtriert und getrocknet. Man erhält so 5,8 g des gewünschten Phenylcarbamats mit dem Schmelzpunkt von 144°-146 °C.

Gemäss diesen Beispielen werden folgende Harnstoffe erhalten :

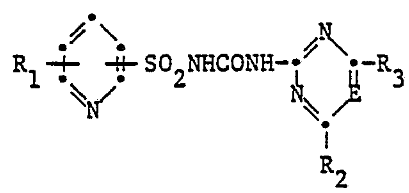

| No. | $R_1$ | X-A | Position $-SO_2-$ | $R_2$ | $R_3$ | E | Smp. °C |
|---|---|---|---|---|---|---|---|
| 1 | H | $2-OC_2H_4OCH_3$ | 3 | $CH_3$ | $OCH_3$ | N | 112-114° |
| 2 | H | $2-OC_2H_4OCH_3$ | 3 | $OCH_3$ | $OCH_3$ | N | |
| 3 | H | $2-OC_2H_4OCH_3$ | 3 | $OCH_3$ | $N(CH_3)_2$ | N | |
| 4 | H | $2-OC_2H_4OCH_3$ | 3 | $OCH_3$ | $OCH_2CF_3$ | N | |
| 5 | H | $2-OC_2H_4OCH_3$ | 3 | $CH_3$ | $CH_3$ | CH | |
| 6 | H | $2-OC_2H_4OCH_3$ | 3 | $CH_3$ | $OCH_3$ | CH | |
| 7 | H | $2-OC_2H_4OCH_3$ | 3 | $OCH_3$ | $OCH_3$ | CH | |
| 8 | H | $2-OC_2H_4OCH_3$ | 3 | $CH_3$ | $OCHF_2$ | CH | 148-150° |
| 9 | H | $2-OCH_2CH=CH_2$ | 3 | $CH_3$ | $OCH_3$ | N | 158-159° |
| 10 | H | $2-OCH_2CH=CH_2$ | 3 | $OCH_3$ | $OCH_3$ | N | 152-154° |
| 11 | H | $2-OCH_2CH=CH_2$ | 3 | $OCH_3$ | $N(CH_3)_2$ | N | |
| 12 | H | $2-OCH_2CH=CH_2$ | 3 | $OCH_3$ | $-OCH_2CF_3$ | N | |
| 13 | H | $2-OCH_2CH=CH_2$ | 3 | $CH_3$ | $CH_3$ | CH | 184-186° |

(Fortsetzung)

| No. | $R_1$ | X-A | Position $-SO_2-$ | $R_2$ | $R_3$ | E | Smp. °C |
|---|---|---|---|---|---|---|---|
| 14 | H | $2-OCH_2CH=CH_2$ | 3 | $CH_3$ | $OCH_3$ | CH | 182-183° |
| 15 | H | $2-OCH_2CH=CH_2$ | 3 | $OCH_3$ | $OCH_3$ | CH | |
| 16 | H | $2-OCH_2CH=CH_2$ | 3 | $CH_3$ | $OCHF_2$ | CH | 158-159° |
| 17 | H | $2-OCH_2-C \equiv CH$ | 3 | $CH_3$ | $OCH_3$ | N | 161-163° |
| 18 | H | $2-OCH_2-C \equiv CH$ | 3 | $OCH_3$ | $OCH_3$ | N | |
| 19 | H | $2-OCH_2-C \equiv CH$ | 3 | $OCH_3$ | $N(CH_3)_2$ | N | |
| 20 | H | $2-OCH_2-C \equiv CH$ | 3 | $OCH_3$ | $OCH_2CF_3$ | N | |
| 21 | H | $2-OCH_2-C \equiv CH$ | 3 | $CH_3$ | $CH_3$ | CH | 188-189° (Z) |
| 22 | H | $2-OCH_2-C \equiv CH$ | 3 | $OCH_3$ | $CH_3$ | CH | 150-152° |
| 23 | H | $2-OCH_2-C \equiv CH$ | 3 | $OCH_3$ | $OCH_3$ | CH | 168-172° |
| 24 | H | $2-OCH_2C \equiv CH$ | 3 | $CH_3$ | $OCHF_2$ | CH | 179-181° |
| 25 | H | $2-OCH_2C(CH_3)=CH_2$ | 3 | $CH_3$ | $OCH_3$ | N | 136-137° |
| 26 | H | $2-OCH_2C(CH_3)=CH_2$ | 3 | $OCH_3$ | $OCH_3$ | N | |
| 27 | H | $2-OCH_2C(CH_3)=CH_2$ | 3 | $OCH_3$ | $N(CH_3)_2$ | N | 153-155° |
| 28 | H | $2-OCH_2C(CH_3)=CH_2$ | 3 | $OCH_3$ | $OCH_2CF_3$ | N | 114-116° |
| 29 | H | $2-OCH_2C(CH_3)=CH_2$ | 3 | $CH_3$ | $CH_3$ | CH | |
| 30 | H | $2-OCH_2C(CH_3)=CH_2$ | 3 | $OCH_3$ | $CH_3$ | CH | 158-159° |
| 31 | H | $2-OCH_2C(CH_3)=CH_2$ | 3 | $OCH_3$ | $OCH_3$ | CH | 146-147° |
| 32 | H | $2-OCH_2C(CH_3)=CH_2$ | 3 | $CH_3$ | $OCHF_2$ | CH | 149-152° |
| 33 | H | $2-OCH_2CH=CHCH_3$ | 3 | $CH_3$ | $OCH_3$ | N | 168-169° |
| 34 | H | $2-OCH_2CH=CHCH_3$ | 3 | $OCH_3$ | $OCH_3$ | N | 140-143° |
| 35 | H | $2-OCH_2CH=CHCH_3$ | 3 | $OCH_3$ | $N(CH_3)_2$ | N | 184-187° |
| 36 | H | $2-OCH_2CH=CHCH_3$ | 3 | $OCH_3$ | $OCH_2CF_3$ | N | 108-111° |
| 37 | H | $2-OCH_2CH=CHCH_3$ | 3 | $CH_3$ | $CH_3$ | CH | 148-150° |
| 38 | H | $2-OCH_2CH=CHCH_3$ | 3 | $OCH_3$ | $CH_3$ | CH | 141-143° |
| 39 | H | $2-OCH_2CH=CHCH_3$ | 3 | $OCH_3$ | $OCH_3$ | CH | 180-184° |
| 40 | H | $2-OCH_2CH=CHCH_3$ | 3 | $CH_3$ | $OCHF_2$ | CH | 146-148° |

(Fortsetzung)

| No. | $R_1$ | X-A | Position $-SO_2-$ | $R_2$ | $R_3$ | E | Smp. °C |
|---|---|---|---|---|---|---|---|
| 41 | H | $2-OC_2H_4Cl$ | 3 | $CH_3$ | $OCH_3$ | N | 168-172° |
| 42 | H | $2-OC_2H_4Cl$ | 3 | $OCH_3$ | $OCH_3$ | N | |
| 43 | H | $2-OC_2H_4Cl$ | 3 | $OCH_3$ | $N(CH_3)_2$ | N | |
| 44 | H | $2-OC_2H_4Cl$ | 3 | $OCH_3$ | $OCH_2CF_3$ | N | |
| 45 | H | $2-OC_2H_4Cl$ | 3 | $CH_3$ | $CH_3$ | CH | |
| 46 | H | $2-OC_2H_4Cl$ | 3 | $CH_3$ | $CH_3$ | CH | |
| 47 | H | $2-OC_2H_4Cl$ | 3 | $OCH_3$ | $OCH_3$ | CH | |
| 48 | H | $2-OC_2H_4Cl$ | 3 | $CH_3$ | $OCHF_2$ | CH | 96-100° |
| 49 | H | $2-OCHF_2$ | 3 | $CH_3$ | $OCH_3$ | N | |
| 50 | H | $2-OCHF_2$ | 3 | $OCH_3$ | $OCH_3$ | N | |
| 51 | H | $2-OCHF_2$ | 3 | $OCH_3$ | $N(CH_3)_2$ | N | |
| 52 | H | $2-OCHF_2$ | 3 | $OCH_3$ | $OCH_2CF_3$ | N | |
| 53 | H | $2-OCHF_2$ | 3 | $CH_3$ | $CH_3$ | CH | |
| 54 | H | $2-OCHF_2$ | 3 | $OCH_3$ | $CH_3$ | CH | |
| 55 | H | $2-OCHF_2$ | 3 | $OCH_3$ | $OCH_3$ | CH | |
| 56 | H | $2-OCHF_2$ | 3 | $CH_3$ | $OCHF_2$ | CH | |
| 57 | H | $2-OCF_2CHF_2$ | 3 | $CH_3$ | $OCH_3$ | N | |
| 58 | H | $2-OCF_2CHF_2$ | 3 | $OCH_3$ | $OCH_3$ | N | |
| 59 | H | $2-OCF_2CHF_2$ | 3 | $OCH_3$ | $N(CH_3)_2$ | N | |
| 60 | H | $2-OCF_2CHF_2$ | 3 | $OCH_3$ | $OCH_2CF_3$ | N | |
| 61 | H | $2-OCF_2CHF_2$ | 3 | $OCH_3$ | $CH_3$ | CH | |
| 62 | H | $2-OCF_2CHF_2$ | 3 | $OCH_3$ | $CH_3$ | CH | |
| 63 | H | $2-OCF_2CHF_2$ | 3 | $OCH_3$ | $OCH_3$ | CH | |
| 64 | H | $2-OCF_2CHF_2$ | 3 | $CH_3$ | $OCHF_2$ | CH | |
| 65 | H | $2-SCHF_2$ | 3 | $CH_3$ | $OCH_3$ | N | |
| 66 | H | $2-SCHF_2$ | 3 | $OCH_3$ | $OCH_3$ | N | |

(Fortsetzung)

| No. | $R_1$ | X-A | Position $-SO_2-$ | $R_2$ | $R_3$ | E |
|---|---|---|---|---|---|---|
| 67 | H | 2-$SCHF_2$ | 3 | $OCH_3$ | $N(CH_3)_2$ | N |
| 68 | H | 2-$SCHF_2$ | 3 | $OCH_3$ | $OCH_2CF_3$ | N |
| 69 | H | 2-$SCHF_2$ | 3 | $CH_3$ | $CH_3$ | CH |
| 70 | H | 2-$SCHF_2$ | 3 | $OCH_3$ | $CH_3$ | CH |
| 71 | H | 2-$SCHF_2$ | 3 | $OCH_3$ | $OCH_3$ | CH |
| 72 | H | 2-$SCHF_2$ | 3 | $CH_3$ | $OCHF_2$ | CH |
| 73 | H | 2-$OC_2H_4OCH_3$ | 3 | $CH_3$ | $OCH_3$ | N |
| 74 | H | 2-$OC_2H_4OCH_3$ | 3 | $OCH_3$ | $OCH_3$ | N |
| 75 | H | 2-$OC_2H_4OCH_3$ | 3 | $OCH_3$ | $N(CH_3)_2$ | N |
| 76 | H | 2-$OC_2H_4OCH_3$ | 3 | $OCH_3$ | $OCH_2CF_3$ | N |
| 77 | H | 2-$OC_2H_4OCH_3$ | 3 | $CH_3$ | $CH_3$ | CH |
| 78 | H | 2-$OC_2H_4OCH_3$ | 3 | $OCH_3$ | $CH_3$ | CH |
| 79 | H | 2-$OC_2H_4OCH_3$ | 3 | $OCH_3$ | $OCH_3$ | CH |
| 80 | H | 2-$OC_2H_4OCH_3$ | 3 | $CH_3$ | $OCHF_2$ | CH |
| 81 | H | 4-$OC_2H_4OCH_3$ | 3 | $CH_3$ | $OCH_3$ | N |
| 82 | H | 4-$OC_2H_4OCH_3$ | 3 | $OCH_3$ | $OCH_3$ | N |
| 83 | H | 4-$OC_2H_4OCH_3$ | 3 | $OCH_3$ | $N(CH_3)_2$ | N |
| 84 | H | 4-$OC_2H_4OCH_3$ | 3 | $OCH_3$ | $OCH_2CF_3$ | N |
| 85 | H | 4-$OC_2H_4OCH_3$ | 3 | $CH_3$ | $CH_3$ | CH |
| 86 | H | 4-$OC_2H_4OCH_3$ | 3 | $OCH_3$ | $CH_3$ | CH |
| 87 | H | 4-$OC_2H_4OCH_3$ | 3 | $OCH_3$ | $OCH_3$ | CH |
| 88 | H | 4-$OC_2H_4OCH_3$ | 3 | $CH_3$ | $OCHF_2$ | CH |
| 89 | H | 4-$OC_2H_4Cl$ | 3 | $CH_3$ | $OCH_3$ | N |
| 90 | H | 4-$OC_2H_4Cl$ | 3 | $OCH_3$ | $OCH_3$ | N |
| 91 | H | 4-$OC_2H_4Cl$ | 3 | $OCH_3$ | $N(CH_3)_2$ | N |
| 92 | H | 4-$OC_2H_4Cl$ | 3 | $OCH_3$ | $OCH_2CF_3$ | N |
| 93 | H | 4-$OC_2H_4Cl$ | 3 | $CH_3$ | $CH_3$ | CH |

(Fortsetzung)

| No. | $R_1$ | X-A | Position $-SO_2-$ | $R_2$ | $R_3$ | E |
|---|---|---|---|---|---|---|
| 94 | H | $4-OC_2H_4Cl$ | 3 | $OCH_3$ | $CH_3$ | CH |
| 95 | H | $4-OC_2H_4Cl$ | 3 | $OCH_3$ | $OCH_3$ | CH |
| 96 | H | $4-OC_2H_4Cl$ | 3 | $CH_3$ | $OCHF_2$ | CH |
| 97 | H | $4-OCF_2$ | 3 | $CH_3$ | $OCH_3$ | N |
| 98 | H | $4-OCF_2$ | 3 | $OCH_3$ | $OCH_3$ | N |
| 99 | H | $4-OCF_2$ | 3 | $OCH_3$ | $N(CH_3)_2$ | N |
| 100 | H | $4-OCF_2$ | 3 | $OCH_3$ | $OCH_2CF_3$ | N |
| 101 | H | $4-OCF_2$ | 3 | $CH_3$ | $CH_3$ | CH |
| 102 | H | $4-OCF_2$ | 3 | $OCH_3$ | $CH_3$ | CH |
| 103 | H | $4-OCF_2$ | 3 | $OCH_3$ | $OCH_3$ | CH |
| 104 | H | $4-OCF_2$ | 3 | $CH_3$ | $OCHF_3$ | CH |
| 105 | H | $4-OCH_2C \equiv CH$ | 3 | $CH_3$ | $OCH_3$ | N |
| 106 | H | $4-OCH_2C \equiv CH$ | 3 | $OCH_3$ | $OCH_3$ | N |
| 107 | H | $4-OCH_2C \equiv CH$ | 3 | $OCH_3$ | $N(CH_3)_2$ | N |
| 108 | H | $4-OCH_2C \equiv CH$ | 3 | $OCH_3$ | $OCH_2CF_3$ | N |
| 109 | H | $4-OCH_2C \equiv CH$ | 3 | $CH_3$ | $CH_3$ | CH |
| 110 | H | $4-OCH_2C \equiv CH$ | 3 | $OCH_3$ | $CH_3$ | CH |
| 111 | H | $4-OCH_2C \equiv CH$ | 3 | $OCH_3$ | $OCH_3$ | CH |
| 112 | H | $4-OCH_2C \equiv CH$ | 3 | $CH_3$ | $OCHF_2$ | CH |
| 113 | $6-CH_3$ | $2-OCH_2CH=CH_2$ | 3 | $CH_3$ | $OCH_3$ | N |
| 114 | $6-CH_3$ | $2-OCH_2CH=CH_2$ | 3 | $OCH_3$ | $OCH_3$ | N |
| 115 | $6-CH_3$ | $2-OCH_2CH=CH_2$ | 3 | $OCH_3$ | $N(CH_3)_2$ | N |
| 116 | $6-CH_3$ | $2-OCH_2CH=CH_2$ | 3 | $OCH_3$ | $OCH_2CF_3$ | N |
| 117 | $6-CH_3$ | $2-OCH_2CH=CH_2$ | 3 | $CH_3$ | $CH_3$ | CH |
| 118 | $6-CH_3$ | $2-OCH_2CH=CH_2$ | 3 | $OCH_3$ | $CH_3$ | CH |
| 119 | $6-CH_3$ | $2-OCH_2CH=CH_2$ | 3 | $OCH_3$ | $OCH_3$ | CH |
| 120 | $6-CH_3$ | $2-OCH_2CH=CH_2$ | 3 | $OCH_3$ | $OCHF_2$ | CH |

(Fortsetzung)

| No. | $R_1$ | X-A | Position $-SO_2-$ | $R_2$ | $R_3$ | E |
|---|---|---|---|---|---|---|
| 121 | 6-$CH_3$ | 2-$OC_2H_4OCH_3$ | 3 | $CH_3$ | $OCH_3$ | N |
| 122 | 6-$CH_3$ | 2-$OC_2H_4OCH_3$ | 3 | $OCH_3$ | $OCH_3$ | N |
| 123 | 6-$CH_3$ | 2-$OC_2H_4OCH_3$ | 3 | $OCH_3$ | $N(CH_3)_2$ | N |
| 124 | 6-$CH_3$ | 2-$OC_2H_4OCH_3$ | 3 | $OCH_3$ | $OCH_2CF_3$ | N |
| 125 | 6-$CH_3$ | 2-$OC_2H_4OCH_3$ | 3 | $CH_3$ | $CH_3$ | CH |
| 126 | 6-$CH_3$ | 2-$OC_2H_4OCH_3$ | 3 | $OCH_3$ | $CH_3$ | CH |
| 127 | 6-$CH_3$ | 2-$OC_2H_4OCH_3$ | 3 | $OCH_3$ | $OCH_3$ | CH |
| 128 | 6-$CH_3$ | 2-$OC_2H_4OCH_3$ | 3 | $CH_3$ | $OCHF_2$ | CH |
| 129 | 6-$CH_3$ | 2-$OCHF_2$ | 3 | $CH_3$ | $OCH_3$ | N |
| 130 | 6-$CH_3$ | 2-$OCHF_2$ | 3 | $OCH_3$ | $OCH_3$ | N |
| 131 | 6-$CH_3$ | 2-$OCHF_2$ | 3 | $OCH_3$ | $N(CH_3)_2$ | N |
| 132 | 6-$CH_3$ | 2-$OCHF_2$ | 3 | $OCH_3$ | $OCH_2CF_3$ | N |
| 133 | 6-$CH_3$ | 2-$OCHF_2$ | 3 | $CH_3$ | $CH_3$ | CH |
| 134 | 6-$CH_3$ | 2-$OCHF_2$ | 3 | $OCH_3$ | $CH_3$ | CH |
| 135 | 6-$CH_3$ | 2-$OCHF_2$ | 3 | $OCH_3$ | $OCH_3$ | CH |
| 136 | 6-$CH_3$ | 2-$OCHF_2$ | 3 | $CH_3$ | $OCHF_2$ | CH |
| 137 | 6-$CH_3$ | 3-$OCHF_2$ | 2 | $CH_3$ | $OCH_3$ | N |
| 138 | 6-$CH_3$ | 3-$OCHF_2$ | 2 | $OCH_3$ | $OCH_3$ | N |
| 139 | 6-$CH_3$ | 3-$OCHF_2$ | 2 | $OCH_3$ | $N(CH_3)_2$ | N |
| 140 | 6-$CH_3$ | 3-$OCHF_2$ | 2 | $OCH_3$ | $OCH_2CF_3$ | N |
| 141 | 6-$CH_3$ | 3-$OCHF_2$ | 2 | $CH_3$ | $OCH_3$ | CH |
| 142 | 6-$CH_3$ | 3-$OCHF_2$ | 2 | $OCH_3$ | $OCH_3$ | CH |
| 143 | 6-$CH_3$ | 3-$OCHF_2$ | 2 | $OCH_3$ | $OCH_3$ | CH |
| 144 | 6-$CH_3$ | 3-$OCHF_2$ | 2 | $CH_3$ | $OCHF_2$ | CH |
| 145 | H | 2-$SO_2C_2H_4OCH_3$ | 3 | $CH_3$ | $OCH_3$ | N |
| 146 | H | 2-$SO_2C_2H_4OCH_3$ | 3 | $OCH_3$ | $OCH_3$ | N |

(Fortsetzung)

| No. | $R_1$ | X-A | Position $-SO_2-$ | $R_2$ | $R_3$ | E | Smp. °C |
|---|---|---|---|---|---|---|---|
| 147 | H | $2-SO_2C_2H_4OCH_3$ | 3 | $OCH_3$ | $N(CH_3)_2$ | N | |
| 148 | H | $2-SO_2C_2H_4OCH_3$ | 3 | $OCH_3$ | $OCH_2CF_3$ | N | |
| 149 | H | $2-SO_2C_2H_4OCH_3$ | 3 | $CH_3$ | $CH_3$ | CH | |
| 150 | H | $2-SO_2C_2H_4OCH_3$ | 3 | $OCH_3$ | $CH_3$ | CH | |
| 151 | H | $2-SO_2C_2H_4OCH_3$ | 3 | $OCH_3$ | $OCH_3$ | CH | |
| 152 | H | $2-SO_2C_2H_4OCH_3$ | 3 | $CH_3$ | $OCHF_2$ | CH | |
| 153 | H | $2-NH_2$ | 3 | $CH_3$ | $OCH_3$ | N | 194-195° |
| 154 | H | $2-NH_2$ | 3 | $OCH_3$ | $OCH_3$ | N | |
| 155 | H | $2-NH_2$ | 3 | $OCH_3$ | $OCH_3$ | CH | |
| 156 | H | $2-NH_2$ | 3 | $OCH_3$ | $CH_3$ | CH | |
| 157 | H | $2-NH_2$ | 3 | $OCHF_2$ | $CH_3$ | CH | |
| 158 | H | $2-NHCH_3$ | 3 | $OCHF_2$ | $CH_3$ | CH | |
| 159 | H | $2-NHCH_3$ | 3 | $OCH_3$ | $CH_3$ | CH | |
| 160 | H | $2-NHCH_3$ | 3 | $CH_3$ | $CH_3$ | CH | |
| 161 | H | $2-NHCH_3$ | 3 | $CH_3$ | $OCH_3$ | N | 147-148° |
| 162 | H | $2-N(CH_3)_2$ | 3 | $CH_3$ | $OCH_3$ | N | 173-175° |
| 163 | H | $2-N(CH_3)_2$ | 3 | $OCH_3$ | $OCH_3$ | N | |
| 164 | H | $2-N(CH_3)_2$ | 3 | $CH_3$ | $OC_2H_5$ | N | |
| 165 | H | $2-N(CH_3)_2$ | 3 | $OCH_3$ | $N(CH_3)_2$ | N | |
| 166 | H | $2-N(CH_3)_2$ | 3 | $OCH_3$ | $CH_3$ | CH | |
| 167 | H | $2-N(CH_3)_2$ | 3 | $OCH_3$ | $OCH_3$ | CH | |
| 168 | H | $2-N(CH_3)_2$ | 3 | $CH_3$ | $OCHF_2$ | CH | |
| 169 | H | $2-N(CH_3)_2$ | 3 | $CH_3$ | $CH_3$ | CH | |
| 170 | H | $2-N(CH_2CH=CH)_2$ | 3 | $CH_3$ | $OCH_3$ | N | 120-122° |
| 171 | H | $2-N(CH_2CH=CH)_2$ | 3 | $CH_3$ | $OCH_3$ | CH | 161-163° |
| 172 | H | $2-N(CH_2CH=CH)_2$ | 3 | $CH_3$ | $OCHF_2$ | CH | 113-122° |
| 173 | H | $2-N(CH_2CH=CH)_2$ | 3 | $OCH_3$ | $OCH_3$ | N | 126-128° |
| 174 | H | $2-N(CH_2CH=CH)_2$ | 3 | $OCH_3$ | $OCH_3$ | CH | 145-149° |
| 175 | H | $2-N(CH_2CH=CH)_2$ | 3 | $CH_3$ | $OC_2H_5$ | N | 165-167° |
| 176 | H | $2-N(CH_2CH=CH)_2$ | 3 | $CH_3$ | $CH_3$ | CH | 180-182° |
| 177 | H | $2-N(CH_2CH=CH)_2$ | 3 | $OCH_3$ | $OCH_2CF_3$ | N | 127-130° |
| 178 | H | 2-Piperidino | 3 | $CH_3$ | $OCH_3$ | N | 177-179° |
| 179 | H | 2-Piperidino | 3 | $CH_3$ | $OCH_3$ | CH | 162-165° |

(Fortsetzung)

| No. | $R_1$ | X-A | Position $-SO_2-$ | $R_2$ | $R_3$ | E | Smp. °C |
|---|---|---|---|---|---|---|---|
| 180 | H | 2-Piperidino | 3 | $OCH_3$ | $CH_3$ | CH | 186-191° |
| 181 | H | 2-Piperidino | 3 | $OCH_3$ | $OCH_3$ | N | 179-181° |
| 182 | H | 2-Morpholino | 3 | $CH_3$ | $OCH_3$ | N | 185-186° |
| 183 | H | 2-Morpholino | 3 | $CH_3$ | $OCH_3$ | CH | 186-189° |
| 184 | H | 2-Morpholino | 3 | $OCH_3$ | $OCH_3$ | CH | 170-173° |
| 185 | H | 2-Morpholino | 3 | $OCH_3$ | $OCH_3$ | N | 100-102° |
| 186 | H | 2-Morpholino | 3 | $OCHF_2$ | $CH_3$ | CH | 184-187° |
| 187 | H | 2-(4'-Methyl-piperazino) | 3 | $OCHF_2$ | $CH_3$ | CH | 172-175° |
| 188 | H | 2-(4'-Methyl-piperazino) | 3 | $OCH_3$ | $CH_3$ | CH | 177-179° |
| 189 | H | 2-(4'-Methyl-piperazino) | 3 | $CH_3$ | $CH_3$ | CH | 159-162° |
| 190 | H | 2-(4'-Methyl-piperazino) | 3 | $CH_3$ | $OCH_3$ | N | 158° |
| 191 | H | 2-(4'-Methyl-piperazino) | 3 | $OCH_3$ | $OCH_3$ | N | |
| 192 | H | 2-(4'-Methyl-piperazino) | 3 | $OCH_3$ | $OCH_3$ | CH | 156-159° |
| 193 | H | 2-Phenoxy | 3 | $CH_3$ | $OCH_3$ | N | 181-182° |
| 194 | H | 2-Phenoxy | 3 | $CH_3$ | $OCH_3$ | CH | 188-189° |
| 195 | H | 2-Phenoxy | 3 | $OCH_3$ | $OCH_3$ | N | |
| 196 | H | 2-Phenoxy | 3 | $OCH_3$ | $N(CH_3)_2$ | N | |
| 197 | H | 2-Phenoxy | 3 | $CH_3$ | $CH_3$ | CH | |
| 198 | H | 2-Phenoxy | 3 | $OCH_3$ | $OCH_3$ | CH | |
| 199 | H | 2-Phenoxy | 3 | $CH_3$ | $OCHF_2$ | CH | |
| 200 | 5-Cl | 2-Phenoxy | 3 | $CH_3$ | $OCH_3$ | N | |
| 201 | 5-Cl | 2-Phenoxy | 3 | $CH_3$ | $OCH_3$ | CH | |
| 202 | H | 2-(2'-Fluorphenoxy) | 3 | $CH_3$ | $OCH_3$ | N | |
| 203 | H | 2-(2'-Fluorphenoxy) | 3 | $CH_3$ | $OCH_3$ | CH | |
| 204 | H | 2-(2'-Fluorphenoxy) | 3 | $OCH_3$ | $OCH_3$ | CH | |
| 205 | H | 2-(3'-Fluorphenoxy) | 3 | $CH_3$ | $OCH_3$ | N | |
| 206 | H | 2-(3'-Fluorphenoxy) | 3 | $CH_3$ | $OCH_3$ | CH | |
| 207 | H | 2-(3'-Fluorphenoxy) | 3 | $OCH_3$ | $OCH_3$ | CH | |
| 208 | H | 2-m-Tolyloxy | 3 | $OCH_3$ | $OCH_3$ | CH | |
| 209 | H | 2-m-Tolyloxy | 3 | $CH_3$ | $OCH_3$ | CH | |
| 210 | H | 2-m-Tolyloxy | 3 | $CH_3$ | $OCH_3$ | N | |
| 211 | H | 2-Phenylthio | 3 | $CH_3$ | $OCH_3$ | N | |

(Fortsetzung)

| No. | $R_1$ | X-A | Position $-SO_2-$ | $R_2$ | $R_3$ | E | Smp. °C |
|---|---|---|---|---|---|---|---|
| 212 | H | 2-Phenylthio | 3 | $CH_3$ | $OCH_3$ | N | |
| 213 | H | 2-Phenylthio | 3 | $CH_3$ | $OCH_3$ | CH | |
| 214 | H | 2-Phenylthio | 3 | $OCH_3$ | $OCH_3$ | CH | |
| 215 | H | 2-Phenylthio | 3 | $CH_3$ | $OCHF_2$ | CH | |
| 216 | H | 3-Phenoxy | 2 | $CH_3$ | $OCH_3$ | N | |
| 217 | H | 3-Phenoxy | 2 | $OCH_3$ | $OCH_3$ | N | |
| 218 | H | 3-Phenoxy | 2 | $CH_3$ | $OCH_3$ | CH | |
| 219 | H | 3-Phenoxy | 2 | $OCH_3$ | $OCH_3$ | CH | |
| 220 | H | 3-Phenoxy | 2 | $CH_3$ | $CH_3$ | CH | |
| 221 | H | 3-Phenoxy | 2 | $CH_3$ | $OCHF_2$ | CH | |
| 222 | H | 3-Phenoxy | 2 | $OCH_3$ | $N(CH_3)_2$ | N | |
| 223 | 5-Cl | 3-Phenoxy | 2 | $CH_3$ | $OCH_3$ | N | |
| 224 | 5-Cl | 3-Phenoxy | 2 | $CH_3$ | $OCH_3$ | CH | |
| 225 | H | 3-(3'-Fluorphenoxy) | 2 | $CH_3$ | $OCH_3$ | CH | |
| 226 | H | 3-(3'-Fluorphenoxy) | 2 | $OCH_3$ | $OCH_3$ | N | |
| 227 | H | 3-(3'-Fluorphenoxy) | 2 | $CH_3$ | $OCH_3$ | N | |
| 228 | H | 3-(3'-Fluorphenoxy) | 2 | $OCH_3$ | $OCH_3$ | CH | |
| 229 | H | 3-(2'-Fluorphenoxy) | 2 | $CH_3$ | $OCH_3$ | CH | |
| 230 | H | 3-(2'-Fluorphenoxy) | 2 | $CH_3$ | $OCH_3$ | N | |
| 231 | H | 3-Phenylthio | 2 | $CH_3$ | $OCH_3$ | N | |
| 232 | H | 3-Phenylthio | 2 | $OCH_3$ | $OCH_3$ | N | |
| 233 | H | 3-Phenylthio | 2 | $CH_3$ | $OCH_3$ | CH | |
| 234 | H | 3-Phenylthio | 2 | $OCH_3$ | $OCH_3$ | CH | |
| 235 | H | $2-S-CH_2-CH=CH_2$ | 3 | $OCH_3$ | $OCHF_2$ | CH | 136-138° |
| 236 | H | $2-S-CH_2-CH=CH_2$ | 3 | $CH_3$ | $OCHF_2$ | CH | |
| 237 | H | $2-S-CH_2-CH=CH_2$ | 3 | $OCH_3$ | Cl | CH | |
| 238 | H | $2-S-CH_2-CH=CH_2$ | 3 | $OCH_3$ | $N(CH_3)_2$ | CH | |
| 239 | H | $2-S-CH_2-CH=CH_2$ | 3 | $OCHF_2$ | $OCHF_2$ | CH | |
| 240 | H | $2-S-CH_2-CH=CH_2$ | 3 | $OCHF_2$ | $OC_2H_5$ | CH | |
| 241 | H | $2-S-CH_2-CH=CH_2$ | 3 | $OCHF_2$ | Cl | CH | |
| 242 | H | $2-S-CH_2-CH=CH_2$ | 3 | $OCH_3$ | $N(CH_3)_2$ | N | |
| 243 | H | $2-S-CH_2-CH=CH_2$ | 3 | $OCH_3$ | $OCH_2CF_3$ | N | |
| 244 | H | $2-NHCH_2CH=CH_2$ | 3 | $CH_3$ | $OCH_3$ | N | 130-133° |
| 245 | H | $2-NHCH_2CH=CH_2$ | 3 | $OCH_3$ | $OCH_3$ | N | 132-134° |
| 246 | H | $2-NHCH_2CH=CH_2$ | 3 | $OCH_3$ | $N(CH_3)_2$ | N | |
| 247 | H | $2-NHCH_2CH=CH_2$ | 3 | $OCH_3$ | $OCH_2CF_3$ | N | |

(Fortsetzung)

| No. | $R_1$ | X-A | Position $-SO_2-$ | $R_2$ | $R_3$ | E | Smp. °C |
|---|---|---|---|---|---|---|---|
| 248 | H | 2-NHCH$_2$CH=CH$_2$ | 3 | $CH_3$ | $CH_3$ | CH | 141-143° |
| 249 | H | 2-NHCH$_2$CH=CH$_2$ | 3 | $CH_3$ | $OCH_3$ | CH | 150-151° |
| 250 | H | 2-NHCH$_2$CH=CH$_2$ | 3 | $OCH_3$ | $OCH_3$ | CH | |
| 251 | H | 2-NHCH$_2$CH=CH$_2$ | 3 | $CH_3$ | $OCHF_2$ | CH | 121-123° |
| 252 | H | 2-NHCH$_2$CH=CH$_2$ | 3 | $OCH_3$ | $OCHF_2$ | CH | |
| 253 | H | 2-NHCH$_2$CH=CH$_2$ | 3 | $OCHF_2$ | $OCHF_2$ | CH | |
| 254 | H | 2-NHCH$_2$CH=CH$_2$ | 3 | $OCHF_2$ | $OC_2H_5$ | CH | |
| 255 | H | 2-NHCH$_2$CH=CH$_2$ | 3 | $OCHF_2$ | Cl | CH | |
| 256 | H | 2-NHCH$_2$CH=CH$_2$ | 3 | $OCH_3$ | Cl | CH | |
| 257 | H | 2-N(CH$_3$)CH$_2$CH=CH$_2$ | 3 | $CH_3$ | $OCH_3$ | N | |
| 258 | H | 2-N(CH$_3$)CH$_2$CH=CH$_2$ | 3 | $CH_3$ | $OC_2H_5$ | N | |
| 259 | H | 2-N(CH$_3$)CH$_2$CH=CH$_2$ | 3 | $OCH_3$ | $OCH_3$ | N | |
| 260 | H | 2-N(CH$_3$)CH$_2$CH=CH$_2$ | 3 | $OCH_3$ | $OCH_2CF_3$ | N | |
| 261 | H | 2-N(CH$_3$)CH$_2$CH=CH$_2$ | 3 | $OCH_3$ | $N(CH_3)_2$ | N | |
| 262 | H | 2-N(CH$_3$)CH$_2$CH=CH$_2$ | 3 | $CH_3$ | $OCH_3$ | CH | |
| 263 | H | 2-N(CH$_3$)C$_2$H$_4$CN | 3 | $CH_3$ | $OCHF_2$ | CH | |
| 264 | H | 2-N(CH$_3$)C$_2$H$_4$CN | 3 | $OCH_3$ | Cl | CH | |
| 265 | H | 2-N(CH$_3$)C$_2$H$_4$CN | 3 | $OCHF_2$ | $OCHF_2$ | CH | |
| 266 | H | 2-N(CH$_3$)C$_2$H$_4$CN | 3 | $CH_3$ | $CH_3$ | CH | |
| 267 | H | 2-Piperidino | 3 | $CH_3$ | $OCHF_2$ | CH | 163-167° |
| 268 | H | 2-piperidino | 3 | $OCH_3$ | Cl | CH | |
| 269 | H | 2-Piperidino | 3 | $CH_3$ | $CH_3$ | CH | 189-192° |
| 270 | H | 2-(4'-Benzyl-piperazino) | 3 | $CH_3$ | $OCH_3$ | N | 200-202° |
| 271 | H | 2-(4'-Benzyl-piperazino) | 3 | $CH_3$ | $OCH_3$ | CH | 176-178° |
| 272 | H | 2-(4'-Benzyl-piperazino) | 3 | $CH_3$ | $OCHF_2$ | N | 230° (Z) |
| 273 | H | 2-OCH$_2$CH=CH$_2$ | 3 | $CH_3$ | $OC_2H_5$ | N | 110-112° |
| 274 | H | 2-OCH$_2$CH=CH$_2$ | 3 | $OCH_3$ | $OCHF_2$ | CH | |
| 275 | H | 2-OCH$_2$CH=CH$_2$ | 3 | $OCHF_2$ | $OCHF_2$ | CH | |
| 276 | H | 2-OCHC(CH$_3$)=CH$_2$ | 3 | $CH_3$ | $OC_2H_5$ | N | |
| 277 | H | 2-OCHC(CH$_3$)=CH$_2$ | 3 | $OCH_3$ | $OCHF_2$ | CH | |
| 278 | H | 2-OCHC(CH$_3$)=CH$_2$ | 3 | $OCHF_3$ | $OCHF_2$ | CH | |
| 279 | H | 2-OCH$_2$CH=CHCH$_3$ | 3 | $CH_3$ | $OC_2H_5$ | N | 131-134° |
| 280 | H | 2-OCH$_2$CH=CHCH$_3$ | 3 | $OCH_3$ | Cl | CH | 157-160° |
| 281 | H | 2-OCH$_2$CH=CHCH$_3$ | 3 | $OCH_3$ | $OCHF_2$ | CH | |

(Fortsetzung)

| No. | $R_1$ | X-A | Position $-SO_2-$ | $R_2$ | $R_3$ | E | Smp. °C |
|---|---|---|---|---|---|---|---|
| 282 | H | $2-OCH_2CH=CHCH_3$ | 3 | $OCHF_2$ | $OCHF_2$ | CH | |
| 283 | H | $2-OCH_2C{\equiv}CH$ | 3 | $CH_3$ | $OC_2H_5$ | N | |
| 284 | H | $2-OCH_2C{\equiv}CH$ | 3 | $OCH_3$ | Cl | CH | |
| 285 | H | $2-OCH_2C{\equiv}CH$ | 3 | $OCH_3$ | $OCHF_2$ | CH | |
| 286 | H | $2-OCHC{\equiv}CH$ | 3 | $OCHF_2$ | $OCHF_2$ | CH | |
| 287 | H | $2-OCH_2CHClCH_2Cl$ | 3 | $CH_3$ | $OCH_3$ | N | 135-138° |
| 288 | H | $2-OCH_2CHClCH_2Cl$ | 3 | $CH_3$ | $OC_2H_5$ | N | |
| 289 | H | $2-OCH_2CHClCH_2Cl$ | 3 | $C_2H_5$ | $OCH_3$ | N | |
| 290 | H | $2-OCH_2CHClCH_2Cl$ | 3 | $OCH_3$ | $OCH_3$ | N | |
| 291 | H | $2-OCH_2CHClCH_2Cl$ | 3 | $OCH_3$ | $OCH_2CF_3$ | N | |
| 292 | H | $2-OCH_2CHClCH_2Cl$ | 3 | $OCH_3$ | $N(CH_3)_2$ | N | |
| 293 | H | $2-OCH_2CHClCH_2Cl$ | 3 | $CH_3$ | $OCH_3$ | CH | |
| 294 | H | $2-OCH_2CHClCH_2Cl$ | 3 | $CH_3$ | $OCHF_2$ | CH | |
| 295 | H | $2-OCH_2CHClCH_2Cl$ | 3 | $CH_3$ | $CH_3$ | CH | |
| 296 | H | $2-OCH_2CHClCH_2Cl$ | 3 | $OCH_3$ | Cl | CH | |
| 297 | H | $2-OCH_2CHClCH_2Cl$ | 3 | $OCH_3$ | $OCH_3$ | CH | |
| 298 | H | $2-OCH_2CHClCH_2Cl$ | 3 | $OCH_3$ | $OCHF_2$ | CH | |
| 299 | H | $2-OCH_2CHClCH_2Cl$ | 3 | $OCH_3$ | $N(CH_3)_2$ | CH | |
| 300 | H | $2-OCH_2CHClCH_2Cl$ | 3 | $OCHF_2$ | $OCHF_2$ | CH | |
| 301 | H | $2-OCH_2CHClCH_2Cl$ | 3 | $OCHF_2$ | $OC_2H_5$ | CH | |
| 302 | H | $2-OCH_2CHClCH_2Cl$ | 3 | $OCHF_2$ | Cl | CH | |
| 303 | H | $2-OCH_2CHBrCH_2Br$ | 3 | $CH_3$ | $OCH_3$ | N | |
| 304 | H | $2-OCH_2CHBrCH_2Br$ | 3 | $CH_3$ | $OC_2H_5$ | N | |
| 305 | H | $2-OCH_2CHBrCH_2Br$ | 3 | $C_2H_5$ | $OCH_3$ | N | |
| 306 | H | $2-OCH_2CHBrCH_2Br$ | 3 | $OCH_3$ | $OCH_3$ | N | |
| 307 | H | $2-OCH_2CHBrCH_2Br$ | 3 | $OCH_3$ | $OCH_2CF_3$ | N | |
| 308 | H | $2-OCH_2CHBrCH_2Br$ | 3 | $OCH_3$ | $N(CH_3)_2$ | N | |
| 309 | H | $2-OCH_2CHBrCH_2Br$ | 3 | $CH_3$ | $OCH_3$ | CH | |
| 310 | H | $2-OCH_2CHBrCH_2Br$ | 3 | $CH_3$ | $OCHF_2$ | CH | |
| 311 | H | $2-OCH_2CHBrCH_2Br$ | 3 | $CH_3$ | $CH_3$ | CH | |
| 312 | H | $2-OCH_2CHBrCH_2Br$ | 3 | $OCH_3$ | Cl | CH | |
| 313 | H | $2-OCH_2CHBrCH_2Br$ | 3 | $OCH_3$ | $OCH_3$ | CH | 148-150° |
| 314 | H | $2-OCH_2CHBrCH_2Br$ | 3 | $OCH_3$ | $OCHF_2$ | CH | |
| 315 | H | $2-OCH_2CHBrCH_2Br$ | 3 | $OCH_3$ | $N(CH_3)_2$ | CH | |
| 316 | H | $2-OCH_2CHBrCH_2Br$ | 3 | $OCHF_2$ | $OCHF_2$ | CH | |
| 317 | H | $2-OCH_2CHBrCH_2Br$ | 3 | $OCHF_2$ | $OC_2H_5$ | CH | |

(Fortsetzung)

| No. | $R_1$ | X-A | Position $-SO_2-$ | $R_2$ | $R_3$ | E | Smp. °C |
|---|---|---|---|---|---|---|---|
| 318 | H | $2-OCH_2CHBrCH_2Br$ | 3 | $OCHF_2$ | Cl | CH | |
| 319 | H | $2-OCH_2CCl(CH_3)CH_2Cl$ | 3 | $CH_3$ | $OCH_3$ | N | |
| 320 | H | $2-OCH_2CCl(CH_3)CH_2Cl$ | 3 | $CH_3$ | $OC_2H_5$ | N | |
| 321 | H | $2-OCH_2CCl(CH_3)CH_2Cl$ | 3 | $C_2H_5$ | $OCH_3$ | N | |
| 322 | H | $2-OCH_2CCl(CH_3)CH_2Cl$ | 3 | $OCH_3$ | $OCH_3$ | N | |
| 323 | H | $2-OCH_2CCl(CH_3)CH_2Cl$ | 3 | $OCH_3$ | $OCH_2CF_3$ | N | |
| 324 | H | $2-OCH_2CCl(CH_3)CH_2Cl$ | 3 | $OCH_3$ | $N(CH_3)_2$ | N | |
| 325 | H | $2-OCH_2CCl(CH_3)CH_2Cl$ | 3 | $CH_3$ | $OCH_3$ | CH | |
| 326 | H | $2-OCH_2CCl(CH_3)CH_2Cl$ | 3 | $CH_3$ | $OCHF_2$ | CH | |
| 327 | H | $2-OCH_2CCl(CH_3)CH_2Cl$ | 3 | $CH_3$ | $CH_3$ | CH | |
| 328 | H | $2-OCH_2CCl(CH_3)CH_2Cl$ | 3 | $OCH_3$ | Cl | CH | |
| 329 | H | $2-OCH_2CCl(CH_3)CH_2Cl$ | 3 | $OCH_3$ | $OCH_3$ | CH | |
| 330 | H | $2-OCH_2CCl(CH_3)CH_2Cl$ | 3 | $OCH_3$ | $OCHF_2$ | CH | |
| 331 | H | $2-OCH_2CCl(CH_3)CH_2Cl$ | 3 | $OCH_3$ | $N(CH_3)_2$ | CH | |
| 332 | H | $2-OCH_2CCl(CH_3)CH_2Cl$ | 3 | $OCHF_3$ | $OCHF_2$ | CH | |
| 333 | H | $2-OCH_2CCl(CH_3)CH_2Cl$ | 3 | $OCHF_2$ | $OC_2H_5$ | CH | |
| 334 | H | $2-OCH_2CCl(CH_3)CH_2Cl$ | 3 | $OCHF_2$ | Cl | CH | |
| 335 | H | $2-OCH_2CBr(CH_3)CH_2Br$ | 3 | $CH_3$ | $OCH_3$ | N | |
| 336 | H | $2-OCH_2CBr(CH_3)CH_2Br$ | 3 | $CH_3$ | $OC_2H_5$ | N | |
| 337 | H | $2-OCH_2CBr(CH_3)CH_2Br$ | 3 | $C_2H_5$ | $OCH_3$ | N | |
| 338 | H | $2-OCH_2CBr(CH_3)CH_2Br$ | 3 | $OCH_3$ | $OCH_3$ | N | |
| 339 | H | $2-OCH_2CBr(CH_3)CH_2Br$ | 3 | $OCH_3$ | $OCH_2CF_3$ | N | |
| 340 | H | $2-OCH_2CBr(CH_3)CH_2Br$ | 3 | $OCH_3$ | $N(CH_3)_2$ | N | |
| 341 | H | $2-OCH_2CBr(CH_3)CH_2Br$ | 3 | $CH_3$ | $OCH_3$ | CH | |
| 342 | H | $2-OCH_2CBr(CH_3)CH_2Br$ | 3 | $CH_3$ | $OCHF_2$ | CH | |
| 343 | H | $2-OCH_2CBr(CH_3)CH_2Br$ | 3 | $CH_3$ | $CH_3$ | CH | |
| 344 | H | $2-OCH_2CBr(CH_3)CH_2Br$ | 3 | $OCH_3$ | Cl | CH | |
| 345 | H | $2-OCH_2CBr(CH_3)CH_2Br$ | 3 | $OCH_3$ | $OCH_3$ | CH | |
| 346 | H | $2-OCH_2CBr(CH_3)CH_2Br$ | 3 | $OCH_3$ | $OCHF_2$ | CH | |
| 347 | H | $2-OCH_2CBr(CH_3)CH_2Br$ | 3 | $OCH_3$ | $N(CH_3)_2$ | CH | |
| 348 | H | $2-OCH_2CBr(CH_3)CH_2Br$ | 3 | $OCHF_2$ | $OCHF_2$ | CH | |
| 349 | H | $2-OCH_2Cbr(CH_3)CH_2Br$ | 3 | $OCHF_2$ | $OC_2H_5$ | CH | |
| 350 | H | $2-OCH_2CBr(CH_3)CH_2Br$ | 3 | $OCHF_2$ | Cl | CH | |
| 351 | H | $2-OCH_2(CHCl)_2CH_3$ | 3 | $CH_3$ | $OCH_3$ | N | |
| 352 | H | $2-OCH_2(CHCl)_2CH_3$ | 3 | $CH_3$ | $OC_2H_5$ | N | |
| 353 | H | $2-OCH_2(CHCl)_2CH_3$ | 3 | $C_2H_5$ | $OCH_3$ | N | |

(Fortsetzung)

| No. | $R_1$ | X-A | Position $-SO_2-$ | $R_2$ | $R_3$ | E | Smp. °C |
|---|---|---|---|---|---|---|---|
| 354 | H | $2-OCH_2(CHCl)_2CH_3$ | 3 | $OCH_3$ | $OCH_3$ | N | |
| 355 | H | $2-OCH_2(CHCl)_2CH_3$ | 3 | $OCH_3$ | $OCH_2CF_3$ | N | |
| 356 | H | $2-OCH_2(CHCl)_2CH_3$ | 3 | $OCH_3$ | $N(CH_3)_2$ | N | |
| 357 | H | $2-OCH_2(CHCl)_2CH_3$ | 3 | $CH_3$ | $OCH_3$ | CH | |
| 358 | H | $2-OCH_2(CHCl)_2CH_3$ | 3 | $CH_3$ | $OCHF_2$ | CH | |
| 359 | H | $2-OCH_2(CHCl)_2CH_3$ | 3 | $CH_3$ | $CH_3$ | CH | 140-143° |
| 360 | H | $2-OCH_2(CHCl)_2CH_3$ | 3 | $OCH_3$ | Cl | CH | |
| 361 | H | $2-OCH_2(CHCl)_2CH_3$ | 3 | $OCH_3$ | $OCH_3$ | CH | |
| 362 | H | $2-OCH_2(CHCl)_2CH_3$ | 3 | $OCH_3$ | $OCHF_2$ | CH | |
| 363 | H | $2-OCH_2(CHCl)_2CH_3$ | 3 | $OCH_3$ | $N(CH_3)_2$ | CH | |
| 364 | H | $2-OCH_2(CHCl)_2CH_3$ | 3 | $OCHF_2$ | $OCHF_2$ | CH | |
| 365 | H | $2-OCH_2(CHCl)_2CH_3$ | 3 | $OCHF_2$ | $OC_2H_5$ | CH | |
| 366 | H | $2-OCH_2(CHCl)_2CH_3$ | 3 | $OCHF_2$ | Cl | CH | |
| 367 | H | $2-OCH_2(CHBr)_2CH_3$ | 3 | $CH_3$ | $OCH_3$ | N | |
| 368 | H | $2-OCH_2(CHBr)_2CH_3$ | 3 | $CH_3$ | $OC_2H_5$ | N | |
| 369 | H | $2-OCH_2(CHBr)_2CH_3$ | 3 | $C_2H_5$ | $OCH_3$ | N | |
| 370 | H | $2-OCH_2(CHBr)_2CH_3$ | 3 | $OCH_3$ | $OCH_3$ | N | |
| 371 | H | $2-OCH_2(CHBr)_2CH_3$ | 3 | $OCH_3$ | $OCH_2CF_3$ | N | |
| 372 | H | $2-OCH_2(CHBr)_2CH_3$ | 3 | $OCH_3$ | $N(CH_3)_2$ | N | |
| 373 | H | $2-OCH_2(CHBr)_2CH_3$ | 3 | $CH_3$ | $OCH_3$ | CH | |
| 374 | H | $2-OCH_2(CHBr)_2CH_3$ | 3 | $CH_3$ | $OCHF_2$ | CH | |
| 375 | H | $2-OCH_2(CHBr)_2CH_3$ | 3 | $CH_3$ | $CH_3$ | CH | |
| 376 | H | $2-OCH_2(CHBr)_2CH_3$ | 3 | $OCH_3$ | Cl | CH | |
| 377 | H | $2-OCH_2(CHBr)_2CH_3$ | 3 | $OCH_3$ | $OCH_3$ | CH | |
| 378 | H | $2-OCH_2(CHBr)_2CH_3$ | 3 | $OCH_3$ | $OCHF_2$ | CH | |
| 379 | H | $2-OCH_2(CHBr)_2CH_3$ | 3 | $OCH_3$ | $N(CH_3)_2$ | CH | |
| 380 | H | $2-OCH_2(CHBr)_2CH_3$ | 3 | $OCHF_2$ | $OCHF_2$ | CH | |
| 381 | H | $2-OCH_2(CHBr)_2CH_3$ | 3 | $OCHF_2$ | $OC_2H_5$ | CH | |
| 382 | H | $2-OCH_2(CHBr)_2CH_3$ | 3 | $OCHF_2$ | Cl | CH | |
| 383 | H | $2-OCH_2CH=CH_2$ | 3 | $OCH_3$ | Cl | CH | 151-161° |
| 384 | H | $2-OCH_2CH=CH_2$ | 3 | $OC_2H_5$ | $OC_2H_5$ | N | 114-116° |
| 385 | H | $2-OCH_2CH=CHCH_3$ | 3 | $OC_2H_5$ | $OC_2H_5$ | N | 106-108° |
| 386 | H | $2-OCH_2C(CH_3)=CH_2$ | 3 | $OC_2H_5$ | $CH_3$ | N | 117-118° |
| 387 | H | $2-OCH_2C(CH_3)=CH_2$ | 3 | $OCH_3$ | Cl | CH | 142-145° |
| 388 | H | $2-OCH_2C(CH_3)=CH_2$ | 3 | $OC_2H_5$ | $OC_2H_5$ | N | 95-98° |

(Fortsetzung)

| No. | $R_1$ | X-A | Position $-SO_2-$ | $R_2$ | $R_3$ | E | Smp. °C |
|---|---|---|---|---|---|---|---|
| 389 | H | $2-N(CH_2CH{=}CH_2)_2$ | 3 | $OCH_3$ | $N(CH_3)_2$ | N | 153-154° |
| 390 | H | $2-N(CH_2CH{=}CH_2)_2$ | 3 | $OCH_3$ | Cl | CH | 140-141° |
| 391 | H | 2-Piperidino | 3 | $OC_2H_5$ | $CH_3$ | N | 152-157° |
| 392 | H | 2-Piperidino | 3 | $OCH_3$ | $N(CH_3)_2$ | N | 210-211° |
| 393 | H | 2-Piperidino | 3 | $OC_2H_5$ | $OC_2H_5$ | N | 121-124° |
| 394 | H | 2-Piperidino | 3 | $OCH_3$ | $OCH_2CF_3$ | N | 133-135° |
| 395 | H | 2-Morpholino | 3 | $OC_2H_5$ | $CH_3$ | N | 107-110° |
| 396 | H | 2-Morpholino | 3 | $OCH_3$ | $N(CH_3)_2$ | N | 153-157° |
| 397 | H | 2-Morpholino | 3 | $OCH_3$ | $OCH_2CF_3$ | N | 92-95° |
| 398 | H | 2-Morpholino | 3 | $CH_3$ | $CH_3$ | CH | 180-183° |
| 399 | H | 2-Morpholino | 3 | $OC_2H_5$ | $OC_2H_5$ | N | 140-143° |
| 400 | H | 2-Morpholino | 3 | $OCH_3$ | Cl | CH | 187-193 |

Gemäss dem Beispiel 1 werden folgende Pyridylsulfonamid-Ausgangsprodukte der Formel II hergestellt.

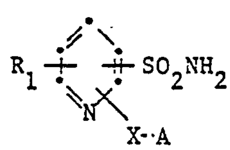

| $R_1$ | X-A | Position $-SO_2NH_2$ | Smp. °C |
|---|---|---|---|
| H | $2-OC_2H_4OCH_3$ | 3 | 102-103° |
| H | $2-OCH_2CH{=}CH_2$ | 3 | 121-121.5 |
| H | $2-OCH_2C{\equiv}CH$ | 3 | 145-47° |
| H | $2-OC(CH_3){=}CH_2$ | 3 | 65-66° |
| H | $2-OCH_2-CH{=}C(CH_3)_2$ | 3 | |
| H | $2-OCH_2CH{=}CHCH_3$ | 3 | 225-226° |
| H | $2-OCHF_2$ | 3 | |
| H | $2-OCH_2CF_3$ | 3 | |
| H | $3-OCHF_2$ | 2 | |
| H | $2-OC_2H_4Cl$ | 3 | |
| H | $2-OCF_2CF_3$ | 3 | |

(Fortsetzung)

| $R_1$ | X-A | Position $SO_2NH_2$ | Smp. °C |
|---|---|---|---|
| H | $2\text{-}OC_2H_4OC_2H_5$ | 3 | |
| H | $2\text{-}OC_2H_4OCH_2CH{=}CH_2$ | 3 | |
| $6\text{-}CH_3$ | $2\text{-}OCH_2CH{=}CH_2$ | 3 | |
| $6\text{-}CH_3$ | $2\text{-}OCH_2C{\equiv}CH$ | 3 | |
| $6\text{-}CH_3$ | $2\text{-}OCHF_2$ | 3 | |
| $6\text{-}CH_3$ | $2\text{-}OC_2H_4Cl$ | 3 | |
| $6\text{-}CH_3$ | $4\text{-}OC_2H_4OCH_3$ | 3 | |
| $6\text{-}CH_3$ | $4\text{-}OCH_2CH{=}CH_2$ | 2 | |
| $6\text{-}CH_3$ | $4\text{-}OCH_2C{\equiv}CH$ | 3 | |
| $6\text{-}CH_3$ | $4\text{-}OC_2H_4Cl$ | 3 | |
| H | $2\text{-}SCH_2CH{=}CH_2$ | 3 | |
| H | $2\text{-}OCF_2CHF_2$ | 3 | |
| H | $2\text{-}SCHF_2$ | 3 | |
| H | $2\text{-}SCH_2C{\equiv}CH$ | 3 | |
| H | $2\text{-}SO_2C_2H_4OCH_3$ | 3 | |
| H | $2\text{-}SO_2CH_2CH{=}CH_2$ | 3 | |
| H | $2\text{-}SO_2CH_2C{\equiv}CH$ | 3 | |
| H | $2\text{-}NH_2$ | 3 | 175-176° |
| H | $2\text{-}NHCH_3$ | 3 | 171-172° |
| H | $2\text{-}N(CH_3)_2$ | 3 | 100-102° |
| H | $2\text{-}N(CH_2CH{=}CH_2)$ | 3 | 90- 91° |
| H | 2-Piperidino | 3 | 155-156° |
| H | 2-Morpholino | 3 | 183-184° |
| H | $2\text{-}OCH(CH_3)CH_2OCH_3$ | 3 | |
| $6\text{-}CH_3$ | $2\text{-}OC_2H_4OCH_3$ | 3 | |
| H | 2-(4'-Benzylpiperazino) | 3 | 164-165° |
| H | 2-(4'-Methylpiperazino) | 3 | 130-134° |

(Fortsetzung)

| $R_1$ | X-A | Position $SO_2NH_2$ | Smp. °C |
|---|---|---|---|
| H | $2-N(CH_3)C_2H_4CN$ | 3 | |
| H | $2-N(C_2H_5)C_2H_4CN$ | 3 | |
| H | 2-Phenoxy | 3 | 176-178° |
| H | $2-OC_2H_4OCH_3$ | 3 | |
| H | $2-OCH_2CH=CH_2$ | 3 | 144-146° |
| H | $2-OCH_2C(CH_3)=CH_2$ | 3 | |
| H | $2-OCH_2CH=CHCH_3$ | 3 | |
| H | $2-S-CH_2CH=CH_2$ | 3 | |
| H | $2-O-CH_2CH=C(CH_3)_2$ | 3 | |
| $6-CH_3$ | $2-O-CH_2=CH_2$ | 3 | |
| H | $2-OCH_2C\equiv CH$ | 3 | |
| H | $N(CH_3)_2$ | 3 | |
| H | $NHCH_2CH=CH_2$ | 3 | 98-99° |

Beispiel 5 : Formulierungsbeispiele für Wirkstoffe der Formel I (% = Gewichtsprozent)

| a) Spritzpulver | a) | b) | c) |
|---|---|---|---|
| Wirkstoff | 20% | 60% | 0,5% |
| Na-Ligninsulfonat | 5% | 5% | 5% |
| Na-Laurylsulfat | 3% | - | - |
| Na-Diisobutylnaphthalinsulfonat | - | 6% | 6% |
| Octylphenolpolyäthylenglykoläther (7-8 Mol AeO) | - | 2% | 2% |
| Hochdisperse Kieselsäure | 5% | 27% | 27% |
| Kaolin | 67% | - | - |
| Natriumchlorid | - | - | 59,5% |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

b) Emulsion-Konzentrat

| | a) | b) |
|---|---|---|
| Wirkstoff | 10% | 1% |
| Octylphenyolpolyäthylenglykoläther (4-5 Mol AeO) | 3% | 3% |
| Ca-Dodecylbenzolsulfonat | 3% | 3% |
| Ricinusölpolyglykoläther (36 Mol AeO) | 4% | 4% |
| Cyclohexamon | 30% | 10% |
| Xylolgemisch | 50% | 79% |

Aus diesem Konzentrat können durch Verdünnen mit Wasser Emulsionen jeder gewünschten Konzentration hergestellt werden.

c) Stäubemittel

| | a) | b) |
|---|---|---|
| Wirkstoff | 0,1% | 1% |
| Talkum | 99,9% | - |
| Kaolin | - | 99% |

Man erhält anwendungsfertige Stäubemittel, indem der Wirkstoff mit dem Träger vermischt und auf einer geeigneten Mühle vermahlen wird.

d) Extruder Granulat

| | a) | b) |
|---|---|---|
| Wirkstoff | 10% | 1% |
| Na-Ligninsulfonat | 2% | 2% |
| Carboxymethylcellulose | 1% | 1% |
| Kaolin | 87% | 96% |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| | |
|---|---|
| Wirkstoff | 3% |
| Polyäthylenglykol (MG 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat

| | a) | b) |
|---|---|---|
| Wirkstoff | 40% | 5% |
| Aethylenglykol | 10% | 10% |
| Nonylphenolpolyäthylenglykoläther (15 Mol AeO) | 6% | 1% |
| Na-Ligninsulfonat | 10% | 5% |
| Carboxymethylcellulose | 1% | 1% |
| 37 %ige wässrige Formaldehyd-Lösung | 0,2 % | 0,2 % |
| Silikonöl in Form einer 75 %igen wässrigen Emulsion | 0,8 % | 0,8 % |
| Wasser | 32 % | 77 % |

Der fein gemahlene Wirkstoff wird mit den Zusatzstoffen innig vermischt.

Man erhält so ein Suspensions-Konzentrat, aus welchem durch Verdünnen mit Wasser Suspensionen jeder gewünschten Konzentration hergestellt werden können.

g) Salzlösung

| | |
|---|---|
| Wirkstoff | 5% |
| Isopropylamin | 1% |
| Octylphenolpolyäthylenglykoläther (78 Mol AeO) | 3% |
| Wasser | 91% |

Beispiel 6 : Herbizidwirkung vor dem Auflaufen der Pflanzen

Kunststofftöpfe werden mit expandiertem Vermiculit (Dichte : 0,135 g/$cm^3$, Wasserabsorptionsvermögen : 0,565 1/1) gefüllt. Nach dem Sättigen des nicht adsorptiven Vermiculits mit einer wässrigen Wirkstoffemulsion in deionisiertem Wasser, die die Wirkstoffe in einer Konzentration von 70,8 ppm enthält, werden Samen der folgenden Pflanzen auf die Oberfläche gesät : Nasturtium officinalis, Agrostis tenuis, Stellaria media und Digitaria sanguinalis. Die Versuchsgefässe werden anschliessend in einer Klimakammer bei 20 °C, einer Beleuchtung von ca. 20 kLux und einer relativen Luftfeuchtigkeit von 70 % gehalten. Während der Keimphase von 4 bis 5 Tagen werden die Töpfe zur Erhöhung der örtlichen Luftfeuchtigkeit mit lichtdurchlässigem Material abgedeckt und mit deionisiertem Wasser gegossen. Nach dem 5. Tag wird dem Giesswasser 0,5 % eines handelsüblichen Flüssigdüngers (® Greenzit) zugesetzt. 12 Tage nach der Aussaat wird der Versuch ausgewertet und die Wirkung auf die Versuchspflanzen nach dem folgenden Massstab bewertet :

1 : Pflanze nicht gekeimt oder total abgestorben
2-3 : sehr starke Wirkung
4-6 : mittlere Wirkung
7-8 : schwache Wirkung
9 : keine Wirkung (wie unbehandelte Kontrolle).

pre-emergente Wirkung :

Konzentration der Wirkstoffemulsion : 70,8 ppm

(Siehe Tabelle Seite 28 f.)

Beispiel 7 : Nachweis der Selektivität bei Vorlaufanwendung

Im Gewächshaus werden Pflanzensamen von dikotylen und monokotylen Unkräutern und Kulturpflanzen in Töpfe von 11 cm Durchmesser gesät. Unmittelbar danach wird die Erdoberfläche mit einer wässrigen Dispersion oder Lösung der Wirkstoffe behandelt. Es werden Konzentrationen von 0,250, 0,125 und 0,06 kg Wirkstoffmengen pro Hektar angewendet. Die Töpfe werden dann im Gewächshaus bei einer Temperatur von 22-25 °C und 50-70 % relativer Luftfeuchtigkeit gehalten. Nach 3 Wochen wird der Versuch ausgewertet und die Wirkung nach dem gleich Massstab, wie im Beispiel 6 angegeben und beurteilt.

(Siehe Tabelle Seite 29 f.)

Beispiel 8 : Nachweis der Herbizidwirkung nach dem Auflaufen der Pflanzen (Kontaktwirkung)

Eine Anzahl Unkräuter und Kulturpflanzen, sowohl monokotyle wie dikotyle, werden nach dem Auflaufen, im 4- bis 6-Blattstadium mit einer wässrigen Wirkstoffdispersion in Dosierungen von 0,5 kg AS/ha gespritzt und dann bei 24° bis 26 °C und 45-60 % relativer Luftfeuchtigkeit gehalten. 15 Tage nach der Behandlung wird der Versuch ausgewertet und nach dem gleichen Massstab wie im pre-emergenten Versuch bewertet.

(Siehe Tabelle Seite 30 f.)

| Testpflanze / Wirkstoff Nr. | Nasturtium | Stellaria | Agrostis | Digitaria |
|---|---|---|---|---|
| 1 | 2 | 2 | 2 | 2 |
| 8 | 1 | 1 | 2 | 2 |
| 9 | 1 | 1 | 1 | 2 |
| 13 | 1 | 1 | 2 | 2 |
| 14 | 1 | 1 | 2 | 1 |
| 16 | 2 | 1 | 3 | 3 |
| 17 | 2 | 1 | 1 | 1 |
| 22 | 1 | 1 | 1 | 1 |
| 24 | 2 | 1 | 2 | 2 |
| 25 | 3 | 3 | 4 | 5 |
| 30 | 1 | 1 | 2 | 2 |
| 33 | 2 | 2 | 3 | 4 |
| 38 | 1 | 1 | 2 | 2 |
| 41 | 3 | 3 | 3 | 7 |
| 48 | 2 | 2 | 4 | 4 |
| 153 | 1 | 1 | 2 | 2 |
| 161 | 2 | 1 | 2 | 2 |
| 162 | 2 | 1 | 2 | 2 |
| Kontrolle (ohne Wirkstoff) | 9 | 9 | 9 | 9 |

Versuchsergebnisse (pre-emergent)

| Wirkung Aufwandmenge kg As/ha | Verb. Nr. 1 | | Verb. Nr. 25 | | Verb. Nr. 33 | | Verb. Nr. 38 | |
|---|---|---|---|---|---|---|---|---|
| Testpflanze | 0.125 | 0.03 | 0.125 | 0.03 | 0.125 | 0.03 | 0.125 | 0.03 |
| Gerste | 7 | 9 | 8 | 9 | 7 | 7 | 2 | 3 |
| Weizen | 9 | 9 | 9 | 9 | 9 | 9 | 2 | 3 |
| Mais | 8 | 9 | 9 | 9 | 9 | 9 | 2 | 9 |
| Reis | 5 | 7 | 7 | 8 | 7 | 9 | 4 | 7 |
| Alopecuvus myos. | 5 | 6 | 4 | 4 | 4 | 4 | 2 | 2 |
| Echinochloa c.g. | 6 | 7 | 5 | 7 | 7 | 9 | 2 | 4 |
| Soja | 5 | 7 | 3 | 4 | 3 | 4 | 4 | 9 |
| Baumwolle | 4 | 7 | 3 | 4 | 3 | 4 | 4 | 8 |
| Amaranthus vet. | 2 | 2 | 2 | 3 | 2 | 2 | 3 | 3 |
| Chenopodium Sp. | 3 | 4 | 4 | 4 | 3 | 6 | 3 | 3 |
| Sinapsis | 2 | 4 | 2 | 2 | 2 | 2 | 2 | 3 |
| Stellaria | 3 | 4 | 2 | 3 | 2 | 2 | 2 | 2 |
| Chrysanth. leuc. | 2 | 4 | 2 | 3 | 2 | 2 | 2 | 2 |
| Galium aparine | 2 | 5 | 2 | 3 | 2 | 2 | 3 | 3 |
| Viola tricolor | 2 | 3 | 2 | 2 | 2 | 2 | 3 | 5 |
| Veronica Sp. | 1 | 2 | 2 | 2 | 2 | 2 | 2 | 2 |

Post-emergente Wirkung :
Aufwandmenge : 0,5 kg Wirksubstanz/Hektar

| Verb. Nr. | Avena Sativa | Setaria italica | Lolium perenne | Solanum lycopers. | Sinapis alba | Stellaria media | Phaseolus vulgaris |
|---|---|---|---|---|---|---|---|
| 1 | 8 | 6 | 3 | 1 | 1 | 2 | 3 |
| 2 | 3 | 5 | 5 | 3 | 2 | 5 | 7 |
| 9 | 5 | 7 | 3 | 2 | 2 | 3 | 3 |
| 14 | 2 | 5 | 3 | 2 | 2 | 4 | 3 |
| 17 | 5 | 4 | 3 | 2 | 2 | 3 | 3 |
| 22 | 3 | 5 | 4 | 2 | 2 | 3 | 3 |

Beispiel 9 : Wuchshemmung bei tropischen Bodendecker-Leguminosen (cover crops)

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70 % relativer Luftfeuchtigkeit und 6 000 lux Kunstlicht, pro Tag 14 Stunden, bei Temperaturen von 27° bei Tag und 21 °C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Formel I behandelten Pflanzen eine deutliche Reduktion des Neuzuwachses (weniger als 20 % des Neuzuwachses bei unbehandelten Kontrollpflanzen) ohne dass dabei die Versuchspflanzen geschädigt wurden.

Beispiel 10 : Wuchsregulierung an Sojabohnen

In Kunststoffbehältern mit einem Erde-Torf-Sandgemisch im Verhältnis 6 : 3 : 1 werden Sojabohnen der Sorte « Hark » angesät und in eine Klimakammer gegeben. Durch optimale Temperaturwahl, Beleuchtung, Düngerzugabe und Bewässerung entwickeln sich die Pflanzen nach ca. 5 Wochen bis zum 5-6 Trifolia-Blattstadium. Zu diesem Zeitpunkt werden die Pflanzen mit der wässrigen Brühe eines Wirkstoffs der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die erfindungsgemässen Wirkstoffe der Formel I eine merkliche Erhöhung der Anzahl und des Gewichts der Schoten im Haupttrieb.

Beispiel 11 : Wuchshemmung bei Getreide

In Kunststofftöpfen mit sterilisierter Erde werden die Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Formel I besprüht. Die Wirkstoffmenge beträgt bis zu 100 g Aktivsubstanz pro Hektar. 21 Tage nach Applikation wird das Wachstum des Getreides beurteilt. Die unbehandelten Pflanzen weisen im Vergleich zu unbehandelten Kontrollen eine Verringerung des Neuzuwachses (60-90 % der Kontrolle), sowie teilweise die Zunahme der Stengeldurchmesser auf.

Beispiel 12 : Wuchshemmung bei Gräsern

In Kunststoffschalten mit Erde-Torf-Sand-Gemisch (6 : 3 : 1) werden die Gräser Lolium perenne, Poa pratensis, Festucca ovina, Dactylis glomerate und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge beträgt umgerechnet bis zu 100 g Aktivsubstanz pro Hektar. 21 Tage nach Applikation wird das Wachstum der Gräser beurteilt.

Die Verbindungen der Formel I bewirken die Reduzierung des Neuzuwachses von 10-30 % im Vergleich zur unbehandelten Kontrolle.

**Patentansprüche**

1. N-Pyridinsulfonyl-N'-pyridinyl- und -triazinylharnstoffe der Formel (I)

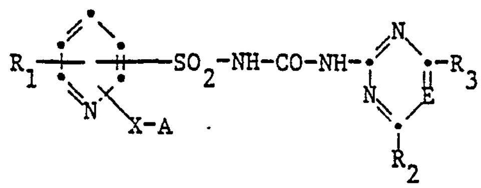

(I)

worin

A einen $C_3$-$C_6$-Alkinylrest, einen durch Halogen, $C_1$-$C_4$-Alkylthio, $C_1$-$C_4$-Alkylsulfinyl, $C_1$-$C_4$-Alkylsulfonyl, $C_1$-$C_4$-Halogenalkoxy, $C_1$-$C_4$-Halogenalkylthio, $C_1$-$C_4$-Halogenalkylsulfinyl oder $C_1$-$C_4$-Halogensulfonyl substituierten $C_1$-$C_6$-Alkylrest oder einen gegebenenfalls durch die aufgezählten Reste substituierten $C_2$-$C_6$-Alkenylenrest, einen Phenylrest, der unsubstituiert oder durch Halogen, Cyano, Nitro, einen Rest X—$C_1$-$C_4$-Alkyl, $C_1$-$C_4$-Alkyl, $C_1$-$C_4$-Halogenalkyl, $C_1$-$C_4$-Alkoxycarbonyl, Amino, Mono- oder Di-($C_1$-$C_4$-alkyl)-amino, Carbamoyl, Mono- oder Di-($C_1$-$C_4$-alkyl)-carbamoyl, Sulfamoyl, Mono- oder Di-($C_1$-$C_4$alkyl)-sulfamoyl substituiert ist oder den Rest

X—A bildet einen Aminorest —$NR_6R_7$,

E die Methingruppe oder Stickstoff,

$R_1$ Wasserstoff, Halogen, einen $C_1$-$C_4$-Alkoxy-, $C_1$-$C_4$-Halogenalkyl, $C_1$-$C_4$-Halogenalkoxy, $C_2$-$C_5$-Alkoxyalkoxy-, $C_1$-$C_5$-Alkylsulfinyl- oder einen $C_1$-$C_5$-Alkylsulfonylrest,

$R_2$ gegebenenfalls durch 1-3 Halogenatome substituiertes $C_1$-$C_3$-Alkyl oder $C_1$-$C_3$-Alkoxy

$R_3$ Wasserstoff, Halogen, eine Aminogruppe, —$NR_4R_5$, gegebenenfalls durch 1-3 Halogenatome substituiertes $C_1$-$C_3$-Alkyl oder gegebenenfalls durch Methoxy, Aethoxy oder 1-3 Halogenatome substituiertes $C_1$-$C_4$-Alkoxy,

$R_4$ Wasserstoff oder Methyl,

$R_5$ Wasserstoff, $C_1$-$C_2$-Alkyl oder Methoxy,

$R_6$ und $R_7$ je einzeln Wasserstoff, $C_1$-$C_4$-Alkyl, $C_3$-$C_6$-Alkenyl, $C_3$-$C_6$-Alkinyl, $C_3$-$C_6$-Alkoxyalkyl, $C_1$-$C_4$-Cyanoalkyl oder

$R_6$ und $R_7$ zusammen mit dem sie verbindenden Stickstoffatom auch einen gesättigten 5-6-gliedrigen Heterocyclus, der auch Sauerstoff, Schwefel oder einen Rest —$NR_8$ enthalten kann,

$R_8$ Wasserstoff, $C_1$-$C_4$-Alkyl oder Benzyl und

X Sauerstoff, Schwefel oder eine Sulfinyl- oder Sulfonylbrücke bedeuten, mit der Massgabe, dass wenn —X—A einen $C_3$-$C_4$-Alkenylsulfid-, -sulfinyl- oder -sulfonylrest und $R_1$ gleichzeitig Wasserstoff, Halogen, Methyl, Methoxy, Trifluoromethyl, Nitro, Cyano oder Amino bedeuten, die Reste $R_2$ auf $C_1$-$C_2$-Alkyl, $C_1$-$C_2$-Alkoxy oder Methoxymethyl und $R_3$ auf Methyl oder Methoxy beschränkt sind,

sowie die Salze dieser Verbindungen.

2. N-Pyridinsulfonyl-N'-pyrimidinyl- und -triazinylharnstoffe gemäss Anspruch 1, Formel I, worin $R_1$ Wasserstoff bedeutet und A, E, $R_2$, $R_3$ und X die im Anspruch 1 gegebene Bedeutung haben.

3. N-Pyridinsulfonyl-N'-pyrimidinyl- und -triazinylharnstoffe gemäss Anspruch 1, Formel I, worin $R_1$ Wasserstoff und X Sauerstoff oder Schwefel bedeuten, während A, E, $R_2$ und $R_3$ die im Anspruch 1 gegebene Bedeutung haben.

4. N-Pyridinsulfonyl-N'-pyrimidinyl- und -triazinylharnstoffe gemäss Anspruch 1, der Formel

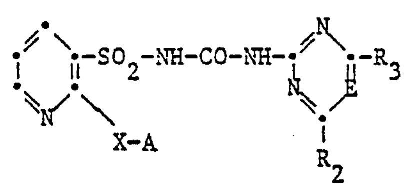

worin A, E, $R_2$, $R_3$ und X die im Anspruch 1 gegebene Bedeutung haben.

5. N-Pyridinsulfonyl-N'-pyrimidinyl- und -triazinylharnstoffe gemäss Anspruch 1 der Formel

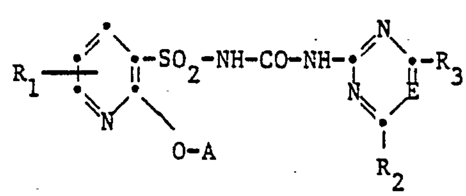

worin A, E, $R_1$, $R_2$ und $R_3$ die im Anspruch 1 gegebene Bedeutung haben.

6. N-Pyridinsulfonyl-N'-pyrimidinylharnstoffe gemäss Anspruch 1 der Formel

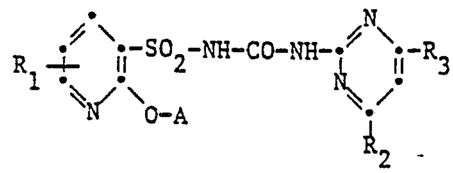

worin A, $R_1$, $R_2$ und $R_3$ die im Anspruch 1 gegebene Bedeutung haben.

7. N-Pyridinsulfonyl-N'-triazinharnstoffe gemäss Anspruch 1 der Formel

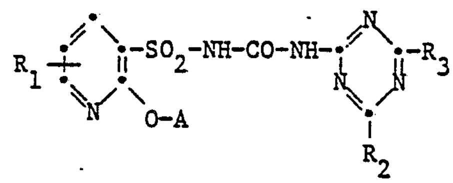

worin A, $R_1$, $R_2$ und $R_3$ die im Anspruch 1 gegebene Bedeutung haben.

8. N-Pyridinylsulfonyl-N'-4-methoxy-6-methyltriazin-2-yl-harnstoffe gemäss Anspruch 1 der Formel

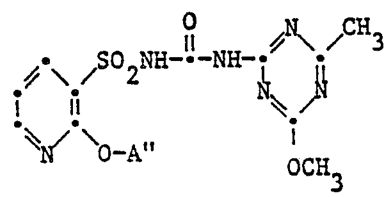

worin A" $C_3$-$C_4$-Alkenyl oder $C_3$-$C_4$-Alkoxyalkyl bedeutet.

9. Verfahren zur Herstellung der N-Pyridinsulfonyl-N'-pyrimidinyl- und -triazinylharnstoffe der Formel I, Anspruch 1, dadurch gekennzeichnet, dass man ein Pyridylsulfonamid der Formel II

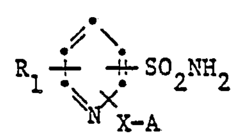

(II)

worin A, $R_1$ und X die im Anspruch 1 gegebene Bedeutung haben, in Gegenwart einer Base als säurebindendes Mittel, mit einem N-Pyrimidinyl- oder N-Triazinylcarbamat der Formel III

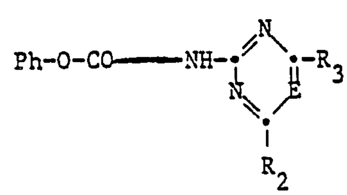

(III)

worin $R_2$, $R_3$, E die im Anspruch 1 gegebene Bedeutung haben und Ph einen gegebenenfalls durch Halogen oder Alkyl substituierten Phenylrest bedeutet.

10. Verfahren zur Herstellung der N-Pyridinsulfonyl-N'-pyrimidinyl- und -triazinylharnstoffe der Formel I, Anspruch 1, dadurch gekennzeichnet, dass man ein Pyridinsulfonylisocyanat der Formel IV

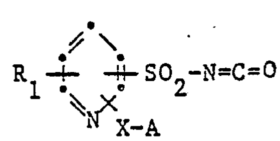

(IV)

worin A, $R_1$ und X die im Anspruch 1 gegebene Bedeutung haben, gegebenenfalls in Gegenwart einer Base mit einem Amin der Formel V umsetzt,

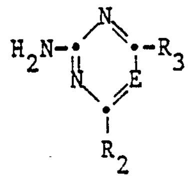

(V)

worin E, $R_2$ und $R_3$ die unter Formel I gegebene Bedeutung haben.

11. Verfahren zur Herstellung der N-Pyridinsulfonyl-N'-pyrimidinyl- und -triazinylharnstoffe der Formel I, Anspruch 1, dadurch gekennzeichnet, dass man ein Pyridinsulfonylcarbamat der Formel VI

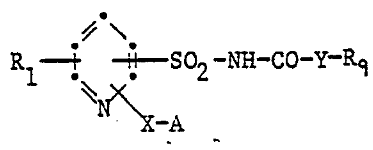

(VI)

worin $R_q$ unsubstituiertes durch Halogen oder $C_1$-$C_4$-Alkyl substituiertes Phenyl, oder $C_1$-$C_4$-Alkyl, $C_2$-$C_4$-Alkoxyalkyl und Y Schwefel oder Sauerstoff bedeuten und A, $R_1$ und X die unter Formel I gegebene Bedeutung haben, in Gegenwart einer Base als säurebindendes Mittel, mit einem Amin der Formel V umsetzt

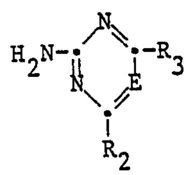

(V)

worin E, $R_2$ und $R_3$ die im Anspruch 1 gegebene Bedeutung haben.

12. Pyridinsulfonamide der Formel II

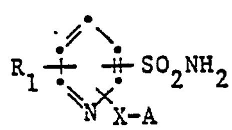

(II)

worin A, $R_1$ und X die im Anspruch 1 gegebene Bedeutung haben.

13. Pyridinsulfonylisocyanate und -thiocyanate der Formel IV

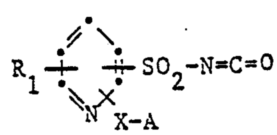

(IV)

worin A, $R_1$ und X die im Anspruch 1 gegebene Bedeutung haben.

14. Pyridinsulfonylcarbamate der Formel VI

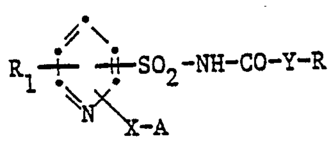

(VI)

worin R unsubstituiert oder durch Halogen oder $C_1$-$C_4$-Alkyl substituiertes Phenyl, $C_1$-$C_4$-Alkyl oder $C_2$-$C_4$-Alkoxyalkyl und Y Sauerstoff oder Schwefel bedeuten und A, $R_1$ und X die im Anspruch 1 gegebene Bedeutung haben.

15. Verfahren zur Herstellung von Additionssalzen der Formel I, gemäss einem der Ansprüche 10 bis 12, dadurch gekennzeichnet, dass man einen Sulfonylharnstoff der Formel I mit einem Amin, einem Alkalimethyl- oder Erdalkalimetallhydroxid oder einer quaternären Ammoniumbase umsetzt.

16. Ein herbizides und den Pflanzenwuchs hemmendes Mittel, dadurch gekennzeichnet, dass es neben Träger- und/oder anderen Zuschlagstoffen als Wirkstoff mindestens einen N-Pyridinsulfonyl-N'-pyrimidinyl- und -triazinylharnstoffe gemäss Anspruch 1, enthält.

17. Die Verwendung der N-Pyridinsulfonyl-N'-pyrimidinyl- und -triazinylharnstoffe gemäss Anspruch 1, oder sie enthaltender Mittel zur Bekämpfung unerwünschten Pflanzenwachstums.

18. Die Verwendung der N-Pyridinsulfonyl-N'-pyrimidinyl- und triazinylharnstoffe gemäss Anspruch 1, oder sie enthaltender Mittel zur Regulierung des Pflanzenwachstums.

19. Die Verwendung der N-Pyridinsulfonyl-N'-pyrimidinyl- und triazinylharnstoffe gemäss Anspruch 1, oder sie enthaltender Mittel, zur selektiven pre- oder postemergenten Bekämpfung von Unkräutern in Nutzpflanzenkulturen.

20. Die Verwendung gemäss Anspruch 20 in Zuckerrohr-, Getreide-, Mais-, Soja-, Reis- und Baumwollkulturen.

21. Die Verwendung gemäss Anspruch 23 in Sojakulturen.

22. Die Verwendung gemäss Anspruch 19 bei Bodendecker-Leguminosen.

**Claims**

1. N-pyridinesulfonyl-N'-pyridinyl- and -triazinylureas of the formula (I)

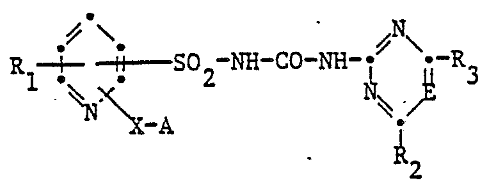
(I)

wherein

A is a $C_3$-$C_6$ alkynyl radical, a $C_1$-$C_6$ alkyl radical which is substituted by halogen, $C_1$-$C_4$ alkylthio, $C_1$-$C_4$-alkylsulfinyl, $C_1$-$C_4$ alkylsulfonyl, $C_1$-$C_4$-haloalkoxy, $C_1$-$C_4$ haloalkylthio, $C_1$-$C_4$ haloalkylsulfinyl or $C_1$-$C_4$ halosulfonyl, or is a $C_2$-$C_6$-alkenylene radical which is unsubstituted or substituted by the radicals listed, or is a phenyl radical which is unsubstituted or substituted by halogen. cyano, nitro, an —X—$C_1$-$C_4$ alkyl, $C_1$-$C_4$-alkyl, $C_1$-$C_4$ haloalkyl, $C_1$-$C_4$ alkoxycarbonyl, amino, mono- or di-($C_1$-$C_4$ alkyl) amino, carbamoyl, mono- or di-($C_1$-$C_4$ alkyl) carbamoyl, sulfamoyl, mono- or di-($C_1$-$C_4$ alkyl) sulfamoyl radical or the radical

X—A forms an amino radical —$NR_6R_7$,

E is the methine group or nitrogen,

$R_1$ is hydrogen, halogen, a $C_1$-$C_4$ alkoxy, $C_1$-$C_4$ haloalkyl, $C_1$-$C_4$ haloalkoxy, $C_2$-$C_5$ alkoxyalkoxy, $C_1$-$C_5$-alkylsulfinyl or $C_1$-$C_5$ alkylsulfonyl radical,

$R_2$ is $C_1$-$C_3$ alkyl which is unsubstituted or substituted by 1 to 3 halogen atoms, or is $C_1$-$C_3$ alkoxy,

$R_3$ is hydrogen, halogen, an amino group —$NR_4R_5$, or $C_1$-$C_3$ alkyl which is unsubstituted or substituted by 1 to 3 halogen atoms, or is $C_1$-$C_4$-alkoxy which is unsubstituted or substituted by methoxy, ethoxy or 1 to 3 halogen atoms,

$R_4$ is hydrogen or methyl,

$R_5$ is hydrogen, $C_1$-$C_2$ alkyl or methoxy,

$R_6$ and $R_7$ are each independently hydrogen, $C_1$-$C_4$ alkyl, $C_3$-$C_6$ alkenyl, $C_3$-$C_6$ alkynyl, $C_3$-$C_6$ alkoxyalkyl, $C_1$-$C_4$-cyanoalkyl, or

$R_6$ and $R_7$ together with the nitrogen atom connecting them also form a saturated 5- or 6-membered heterocycle which may also contain oxygen, sulfur or an —$NR_8$— radical,

$R_8$ is hydrogen, $C_1$-$C_4$ alkyl or benzyl, and

X is oxygen, sulfur or a sulfinyl or sulfonyl bridge, with the proviso that if —X—A forms a $C_3$-$C_4$ alkenylsulfide, -sulfinyl or -sulfonyl radical and $R_1$ is simultaneously hydrogen, halogen, methyl, methoxy, trifluoromethyl, nitro, cyano or amino, then the radical $R_2$ must be $C_1$-$C_2$ alkyl, $C_1$-$C_2$ alkoxy or methoxymethyl and the radical $R_3$ methyl or methoxy,

and the salts of these compounds.

2. N-pyridinesulfonyl-N'-pyrimidinyl- and -triazinylureas according to claim 1, formula I, wherein $R_1$ is hydrogen and A, E, $R_2$, $R_3$ and X are as defined in claim 1.

3. N-pyridinesulfonyl-N'-pyrimidinyl- and -triazinylureas according to claim 1, formula I, wherein $R_1$ is hydrogen and X is oxygen of sulfur, while A, E, $R_2$ and $R_3$ are as defined in claim 1.

4. N-pyridinesulfonyl-N'-pyrimidinyl- and -triazinylureas according to claim 1, of the formula

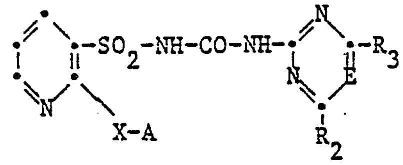

wherein A, E, $R_2$, $R_3$ and X are as defined in claim 1.

5. N-pyridinesulfonyl-N'-pyrimidinyl- and -triazinylureas according to claim 1, of the formula

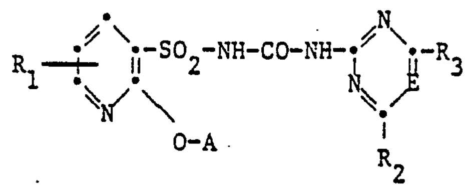

wherein A, E, $R_1$, $R_2$ and $R_3$ are as defined in claim 1.

6. N-pyridinesulfonyl-N'-pyrimidinylureas according to claim 1 of the formula

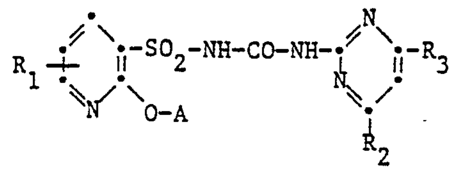

wherein A, $R_1$, $R_2$ and $R_3$ are as defined in claim 1.

7. N-pyridinesulfonyl-N'-triazineureas according to claim 1, of the formula

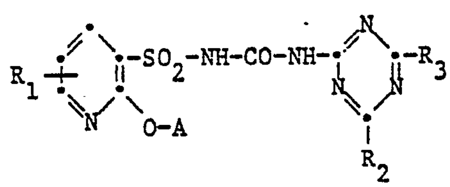

wherein A, $R_1$, $R_2$ and $R_3$ are as defined in claim 1.

8. N-pyridinesulfonyl-N'-4-methoxy-6-methyltriazin-2-ylureas according to claim 1, of the formula

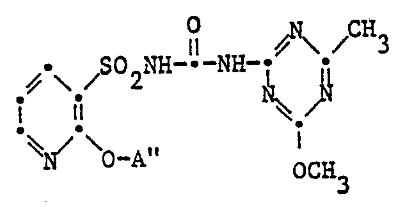

wherein A'' is $C_3$-$C_4$ alkenyl or $C_3$-$C_4$ alkoxyalkyl.

9. Process for the preparation of the N-pyridinesulfonyl-N'-pyrimidinyl- and -triazinylureas of the formula I, claim 1, characterised in that a pyridyl-sulfonamide of the formula II

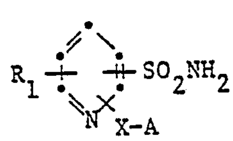

(II)

wherein A, $R_1$ and x are as defined in claim 1, with an N-pyrimidinyl- or N-triazinylcarbamate of the formula III

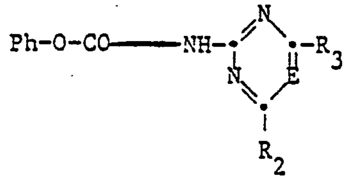

(III)

wherein $R_2$, $R_3$ and E are as defined in claim 1 and Ph is a phenyl radical which is unsubstituted or substituted by halogen or alkyl, in the presence of a base as an acid-binding agent.

10. Process for the preparation of the N-pyridine-sulfonyl-N'-pyrimidinyl- and -triazinylureas of the formula I, claim 1, characterised in that a pyridine-sulfonyl isocyanate of the formula IV

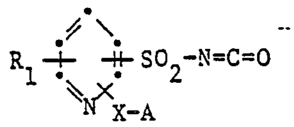 (IV)

wherein A, $R_1$ and X are as defined in claim 1, is reacted with an amine of the formula V

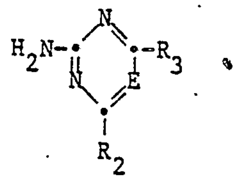 (V)

wherein E, $R_2$ and $R_3$ are as defined under formula I, optionally in the presence of a base.

11. Process for the preparation of the N-pyridine-sulfonyl-N'-pyrimidinyl and -triazinylureas of the formula I, claim 1, characterised in that a pyridine-sulfonylcarbamate of the formula VI

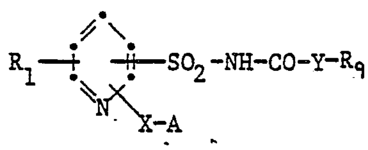 (VI)

wherein $R_q$ is unsubstituted phenyl substituted by halogen or $C_1$-$C_4$ alkyl, or is $C_1$-$C_4$ alkyl or $C_2$-$C_4$-alkoxyalkyl, Y is sulfur or oxygen, and A, $R_1$ and X are as defined under formula I, is reacted with an amine of the formula V

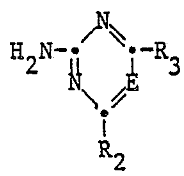 (V)

wherein E, $R_2$ and $R_3$ are as defined in claim 1, in the presence of a base as acid-binding agent.

12. Pyridinesulfonamides of the formula II

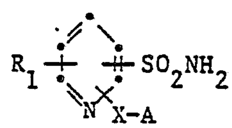 (II)

wherein A, $R_1$ and X are as defined in claim 1.

13. Pyridinesulfonyl isocyanates and isothiocyanates of the formula IV

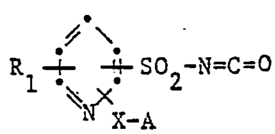 (IV)

wherein A, $R_1$ and X are as defined in claim 1.

14. Pyridinesulfonylcarbamates of the formula VI

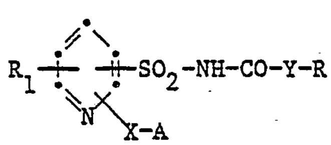 (VI)

wherein R is phenyl which is unsubstituted or substituted by halogen or $C_1$-$C_4$ alkyl, or is $C_1$-$C_4$ alkyl or $C_2$-$C_4$ alkoxyalkyl, Y is oxygen or sulfur, and A, $R_1$ and X are as defined in claim 1.

15. Process for the preparation of addition salts of the formula I according to any one of claims 10 to

12, characterised in that a sulfonylurea of the formula I is reacted with an amine, an alkali methyl hydroxide or alkaline earth metal hydroxide or with a quaternary ammonium base.

16. A herbicidal and plant growth inhibiting agent, characterised in that it contains as active ingredient at least one N-pyridinesulfonyl-N'-pyrimidinyl- and -triazinylureas according to claim 1, together with carriers and/or other adjuvants.

17. The use of the N-pyridinesulfonyl-N'-pyrimidinyl- and -triazinylureas according to claim 1, or of agents containing them, for controlling undesired plant growth.

18. The use of the N-pyridinesulfonyl-N'-pyrimidinyl- and -triazinylureas according to claim 1, or of agents containing them, for regulating plant growth.

19. The use of the N-pyridinesulfonyl-N'-pyrimidinyl- and -triazinylureas according to claim 1, or of agents containing them, for selectively controlling weeds pre- or postemergence in crops of useful plants.

20. The use according to claim 20, in crops of sugar cane, cereals, maize, soybeans, rice and cotton.

21. The use according to claim 23, in crops of soybeans.

22. The use according to claim 19, in the case of cover crop leguminosae.

**Revendications**

1. N-Pyridinsulfonyl-N'pyridinyl- et -triazinylurées de Formule (I)

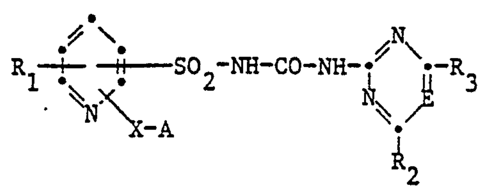

(I)

où

A représente un radical Alcynyle en $C_3$-$C_6$, un radical Alcoyle en $C_1$-$C_6$ substitué par un halogène, Alcoxy en $C_1$-$C_4$, Alcoylthio en $C_1$-$C_4$, Alcoylsulfinyle en $C_1$-$C_4$, Alcoylsulfonyle en $C_1$-$C_4$, Halogènealcoxy en $C_1$-$C_4$, Halogenalcoylthio en $C_1$-$C_4$, Halogenalcoylsulfinyle en $C_1$-$C_4$ ou Halogènalcoylsulfonyle en $C_1$-$C_4$ ou un radical Alcényle en $C_2$-$C_6$ éventuellement substitué par les radicaux énumérés, un radical Phényle, qui est non substitué ou substitué par un Halogène, Cyano, Nitro, Alcoyle en $C_1$-$C_4$, Halogènalcoyle en $C_1$-$C_4$, un radical —X—Alcoyl en $C_1$-$C_4$, Alcoxycarbonyle en $C_1$-$C_4$, Amino, Mono- ou Di-(Alcoyle en $C_1$-$C_4$) amino, Carbamoyl, Mono- ou Di-(Alcoyle en $C_1$-$C_4$) carbamoyle, Sulfamoyle, Mono- ou Di-(Alcoyle en $C_1$-$C_4$) sulfamoyle, ou le radical

X—A forme un radical Amino —$NR_6R_7$,

E représente le groupe méthine ou un azote,

$R_1$ représente un hydrogène, halogène, un radical alcoyl en $C_1$-$C_4$, alcoxy en $C_1$-$C_4$, halogènalcoyle en $C_1$-$C_4$, halogènalcoxy en $C_1$-$C_4$, alcoxyalcoxy en $C_2$-$C_5$, alcoylthio en $C_1$-$C_5$, alcoylsulfinyle en $C_1$-$C_5$ ou alcoylsulfonyle en $C_1$-$C_5$,

$R_2$ représente un alcoyle en $C_1$-$C_3$ éventuellement substitué par de 1 à 3 atomes d'halogène, ou un alcoxy en $C_1$-$C_3$,

$R_3$ représente un hydrogène, halogène, un groupe amino —$NR_4R_5$, un alcoyle en $C_1$-$C_3$ éventuellement substitué par de 1 à 3 atomes d'halogène ou un alcoxy en $C_1$-$C_4$ éventuellement substitué par un méthoxy, éthoxy ou de 1 à 3 atomes d'halogène,

$R_4$ représente un hydrogène ou un méthyle,

$R_5$ représente un hydrogène, alcoyle en $C_1$-$C_2$ ou méthoxy

$R_6$ et $R_7$ représentent chacun isolément un hydrogène, alcoyle en $C_1$-$C_4$, alcényle en $C_3$-$C_6$, alcynyl en $C_3$-$C_6$, alcoxyalcoyle en $C_3$-$C_6$, cyanoalcoyl en $C_1$-$C_4$ ou

$R_6$ et $R_7$ représentent également avec l'atome d'azote qui les relie un hétérocycle saturé à 5-6 chaînons qui peut également contenir un oxygène, un soufre ou un radical —$NR_8$—,

$R_8$ représente un hydrogène, alcoyle en $C_1$-$C_4$ ou benzyle, et

X représente un oxygène, un soufre, un pont sulfinyl ou sulfonyle, avec la précision que lors —X—A représente un radical alcynyle en $C_3$-$C_4$ -sulfure, -sulfinyle ou -sulfonyle et $R_1$ représente simultanément un hydrogène, halogène, méthyle, méthoxy, trifluorométhyle, nitro, cyano ou amino, les radicaux $R_2$ se limitent à alcoyle en $C_1$-$C_2$, alcoxy en $C_1$-$C_2$ ou méthoxyméthyle et $R_3$ à méthyl ou méthoxy, ainsi que les sels de ces composés.

2. N-Pyridinsulfonyl-N'-pyrimidinyl- et -triazinylurée selon la revendication 1, de formule I, où $R_1$ représente un hydrogène et A, E, $R_2$, $R_3$ et X ont la signification donnée dans la revendication 1.

3. N-Pyridinsulfonyl-N'-pyrimidinyl- et -triazinylurée selon la revendication 1, de formule I, où $R_1$ représente un hydrogène et X un oxygène ou un soufre, tandis que A, E, $R_2$ et $R_3$ ont la signification donnée dans la revendication 1.

4. N-Pyridinsulfonyl-N'-pyrimidinyl- et -triazinylurée selon la revendication 1, de formule

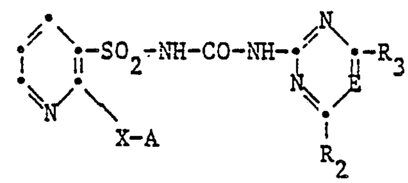

où A, E, $R_2$, $R_3$ et X ont la signification donnée dans la revendication 1.

5. N-Pyridinsulfonyl-N'-pyrimidinyl- et -triazinylurée selon la revendication 1, de formule

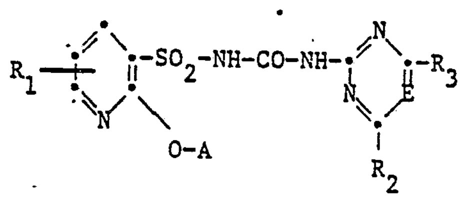

où A, E, $R_1$, et $R_3$ ont la signification donnée dans la revendication 1.

6. N-Pyridinsulfonyl-N'-pyrimidinylurée selon la revendication 1, de formule

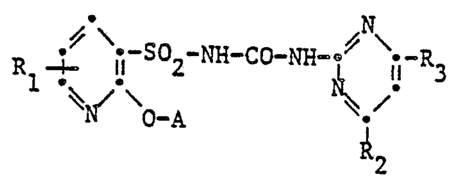

où A, $R_1$, $R_2$ et $R_3$ ont la signification donnée dans la revendication 1.

7. N-Pyridinsulfonyl-N'-triazinurée selon la revendication 1 de formule

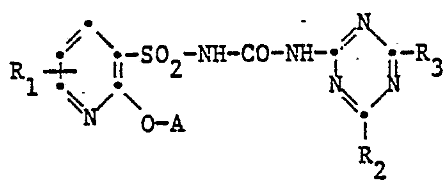

où A, $R_1$, $R_2$ et $R_3$ ont la signification donnée dans la revendication 1.

8. N-Pyridinsulfonyl-N'-4-methoxy-6-methyltriazin-2-yl-urées selon la revendication 1 de formule

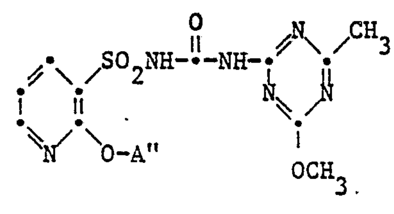

où A" représente un Alcényl en $C_3$-$C_4$ ou un Alcoxyalcoyl en $C_3$-$C_4$.

9. Procédé de préparation de N-(Pyridinsulfonyl-N'-pyrimidinyl- et -triazinylurée de formule I de la revendication 1, caractérisé en ce qu'on fait réagir un Pyridylsufonamide de formule II

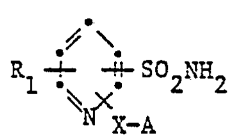 (II)

où A, $R_1$ et X ont la signification donnée dans la revendication 1, en présence d'une base comme agent liant d'acide, avec un N-Pyrimidinyl- ou N-Triazinylcarbamate de formule III

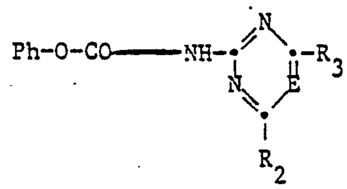 (III)

où $R_2$, $R_3$, E ont la signification donnée dans la revendication 1 et Ph représente un radical Phényle éventuellement substitué par un Halogène ou un Alcoyle.

10. Procédé de préparation des N-Pyridinsulfonyl-N'-pyrimidinyl- et -triazinylurées de formule I de la revendication 1, caractérisé en ce qu'on fait réagir un Pyridinsulfonylisocyanate de formule IV

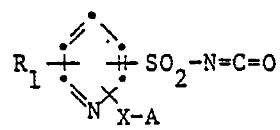 (IV)

où A, $R_1$ et X ont la signification donnée dans la revendication 1, éventuellement en présence d'une base, avec une amine de formule V

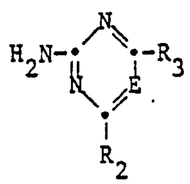 (V)

où E, $R_2$ et $R_3$ ont la signification donnée sous la formule I.

11. Procédé de préparation des N-pyridinsulfonyl-N'-pyrimidinyl- et -triazinylurées de formule I de la revendication 1, caractérisé en ce qu'on fait réagir un Pyridinsulfonylcarbamate de formule VI

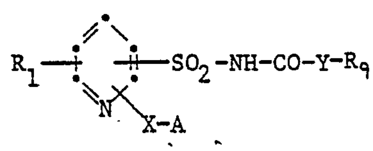 (VI)

où $r_q$ représente un Phényle non substitué ou substitué par un Halogène ou un Alcoyle en $C_1$-$C_4$, ou un Alcoyle en $C_1$-$C_4$, un Alcoxyalcoyle en $C_2$-$C_4$ et Y représente un soufre ou un oxygène et A, $R_1$ et X ont la signification donnée sous la formule I, en présence d'une base comme agent liant d'acide, avec une amine de formule V

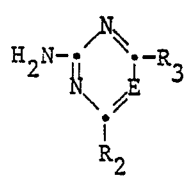 (V)

où E, $R_2$ et $R_3$ ont la signification donnée dans la revendication 1.

12. Pyridinsulfonamide de formule II

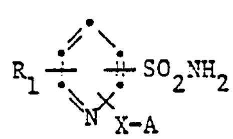 (II)

où A, $R_1$ et X ont la signification donnée dans la revendication 1.

13. Pyridinesulfonylisocyanate et -thiocyanate de formule IV

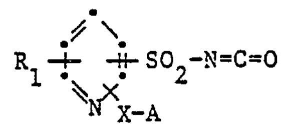

(IV)

où A, $R_1$ et X ont la signification donnée dans la revendication 1.

14. Pyridinsulfonylcarbamates de formule VI

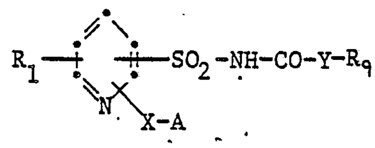

(VI)

où R représente un Phenyl non substitué ou substitué par un Halogène ou un Alcoyle en $C_1$-$C_4$, un Alcoyle en $C_1$-$C_4$ ou un Alcoxyalcoyle en $C_2$-$C_4$ et Y représente un oxygène ou un soufre et A, $R_1$ et X ont la signification donnée dans la revendication 1.

15. Procédé de préparation de sels d'addition d'acides de Formule I selon l'une des revendications 10 à 12, caractérisé en ce qu'on fait réagir une Sulfonylurée de Formule I avec une amine, un Hydroxyde de métal alcalin ou de métal alcalino-terreux ou une base d'ammonium quaternaire.

16. Herbicide et agent inhibant la croissance des plantes, caractérisé en ce qu'il contient outre des supports et/ou d'autres additifs comme matière active au moins une N-Pyridinsulfonyl-N'-pyrimidinyl- et -triazinylurée selon la revendication 1.

17. Application des N-Pyridinsulfonyl-N'-pyrimidinyl- et -triazinylurées selon la revendication 1 ou des agents qui les contiennent à la lutte contre la croissance végétale indésirable.

18. Application des N-Pyridinsulfonyl-N'-pyrimidinyl- et -triazinylurées selon la revendication 1, ou des agents qui les contiennent, à la régulation de la croissance végétale.

19. Application des N-Pyridinsulfonyl-N'-pyrimidinyl- et -triazinylurées selon la revendication 1, ou des agents qui les contiennent, à la lutte sélective en pré ou en post levée contre les mauvaises herbes dans les cultures de plantes utiles.

20. Application selon la revendication 20 dans les cultures de betteraves sucrières, de céréales, de maïs, de soja, de riz et de coton.

21. Application selon la revendication 23 dans les cultures de soja.

22. Application selon la revendication 19 dans les légumineuses protectrices du sol.